# EUROPEAN PATENT APPLICATION

(11) **EP 4 487 854 A1**
(43) Date of publication of application: **08.01.2025**
(21) Application number: 23763593.3
(22) Date of filing: 03.03.2023
(51) Int. Cl.: A61K 31/713, A61K 9/127, A61K 31/7088, A61K 48/00, A61P 1/16, A61P 9/04, A61P 9/10, A61P 13/12, A61P 35/00, A61P 43/00, C12N 15/113

(54) **PHARMACEUTICAL COMPOSITION FOR TREATMENT OR PREVENTION OF FIBROTIC DISEASE**

(30) Priority: 03.03.2022 JP 2022032915
(71) Applicant: Kyushu University, National University Corporation, Nishi-ku Fukuoka-shi Fukuoka 819-0395 (JP)
(72) Inventor: NAKAYA, Michio, Fukuoka-shi, Fukuoka 819-0395 (JP)
(74) Representative: Weickmann & Weickmann PartmbB
(86) International application number: PCT/JP2023/008195
(87) International publication number: WO 2023/167336

(57) **Abstract**

The pharmaceutical composition for treating or preventing a fibrotic disease of the present invention contains a nucleic acid that inhibits the expression of mRNA encoding P4HA3. The fibrotic disease may be at least one disease selected from the group consisting of fibrosis, liver cirrhosis, renal failure, myocardial infarction, and cancer. The present invention provides a method for preventing or treating a fibrotic disease, which includes administering an effective amount of the pharmaceutical composition of the present invention to a subject suffering from or at risk of suffering from a fibrotic disease.

## Description

### [Technical Field]

The present invention relates to a pharmaceutical composition comprising a nucleic acid that inhibits the expression of mRNA encoding the alpha 3 subunit of prolyl 4-hydroxylase (P4HA3), and more specifically, to a pharmaceutical composition comprising the nucleic acid for treating or preventing at least one fibrotic disease selected from the group consisting of pulmonary and other organ fibrosis, liver cirrhosis, renal failure, myocardial infarction, and cancer.

### [Background Art]

Fibrosis refers to a state in which fibrous proteins such as collagen are excessively accumulated in an organ. As explained in detail in the detailed description of the present specification, in the present specification, all diseases characterized by physical hardening of an organ caused by the overproduction of fibrous proteins are called fibrotic diseases. In fibrotic diseases, fibrotic proteins that are excessively accumulated in an organ are called fibrosis-related factors. Fibrotic diseases have in common that myofibroblasts are the main agents that overproduce fibrosis-related factors, regardless of the organ. Fibrotic diseases are involved in approximately 45% of all causes of death in developed countries (Non Patent Literature 1). Conventionally, attempts to treat fibrotic diseases using nucleic acid drugs targeting a growth factor involved in the differentiation of myofibroblasts (TGFβ, Non Patent Literature 2) or a collagen-specific molecular chaperone (HSP47, Patent Literature 1, Non Patent Literatures 3 and 4) have been known. However, the target genes of these conventional candidate therapeutic drugs for fibrotic diseases do not have high expression specificity for myofibroblasts, and the effect of suppressing the expression of fibrosis-related factors is also limited.

### [Citation List]

### [Patent Literature]

[Patent Literature 1]
   JP-B-6433079

### [Non Patent Literature]

[Non Patent Literature 1]
   Pellicoro, A., et al. (2014) Nat. Rev. Immunol., 14(3):181-94.
[Non Patent Literature 2]
   D'Alessandro-Gabazza1, C.N., et al. (2012) Am J Respir Cell Mol Biol Vol 46, Iss. 3, pp 397-406.
[Non Patent Literature 3]
   Ishiwatari, H., et al. (2013) Gut, 62(9), 1328-1339.
[Non Patent Literature 4]
   Liu, Y., et al. (2021) ERJ Open Res, 7: 00733-2020.

### [Summary of Invention]

### [Technical Problem]

The object of the present invention is to provide a novel pharmaceutical composition for treating or preventing fibrotic diseases, which has higher expression specificity for target genes in myofibroblasts and has a higher effect of suppressing the expression of fibrosis-related factors than conventional therapeutic drug candidates for fibrotic diseases. To this end, it is necessary to identify genes that are specifically expressed only in myofibroblasts and are directly involved in the overproduction of fibrosis-related factors. There is a great demand for pharmaceutical compositions that can be expected to have a higher therapeutic or preventive effect in more fibrotic diseases.

### [Solution to Problem]

As a result of diligent efforts, the present inventors have searched for genes that are specifically expressed in myofibroblasts, and discovered that the alpha 3 subunit (P4HA3), a specific isoform of the alpha subunit of prolyl 4-hydroxylase, is specifically expressed in myofibroblasts as a molecular target that can prevent the overproduction of extracellular matrix proteins by myofibroblasts, and that inhibition of the expression of this gene suppresses the production of collagen and other fibrosis-related proteins, resulting in the improvement of fibrotic diseases, thereby completing the present invention.

The present invention provides a pharmaceutical composition for treating or preventing a fibrotic disease. The pharmaceutical composition of the present invention includes a nucleic acid that inhibits the expression of mRNA encoding the alpha 3 subunit of prolyl 4-hydroxylase (hereinafter referred to as "P4HA3").

In the pharmaceutical composition of the present invention, the fibrotic disease may be at least one disease selected from the group consisting of fibrosis in the lung, skin, or other organs, liver cirrhosis, renal failure, myocardial infarction, and cancer.

In the pharmaceutical composition of the present invention, the nucleic acid may have a nucleotide sequence that is complementary to at least a portion of the mRNA encoding P4HA3.

In the pharmaceutical composition of the present invention, the nucleic acid may be an siRNA or an antisense nucleic acid.

In the pharmaceutical composition of the present invention, the siRNA may be an siRNA consisting of an oligonucleotide containing at least 15 consecutive sequences of any one of the sequences of SEQ ID NOs: 9 to 11, and an oligonucleotide containing a sequence complementary to at least 15 consecutive sequences of the oligonucleotide.

In the pharmaceutical composition of the present invention, the siRNA may be a pair of an oligonucleotide containing the sequence of SEQ ID NO:5 and an oligonucleotide containing the sequence of SEQ ID NO:6, or a pair of an oligonucleotide containing the sequence of SEQ ID NO:7 and an oligonucleotide containing the sequence of SEQ ID NO:8.

In the pharmaceutical composition of the present invention, the siRNA may be a pair of an oligonucleotide consisting of the sequence of SEQ ID NO:1 and an oligonucleotide consisting of the sequence of SEQ ID NO:2, or a pair of an oligonucleotide consisting of the sequence of SEQ ID NO:3 and an oligonucleotide consisting of the sequence of SEQ ID NO:4.

The pharmaceutical composition of the present invention may include a nucleic acid delivery carrier.

In the pharmaceutical composition of the present invention, the nucleic acid delivery carrier may be a liposome or a lipid nanoparticle.

In the pharmaceutical composition of the present invention, the liposome may be a liposome that specifically adsorbs to myofibroblasts and encapsulates the nucleic acid.

The pharmaceutical composition of the present invention may further include a drug P other than the nucleic acid that inhibits the expression of mRNA encoding P4HA3.

The present invention provides a method for preventing or treating a fibrotic disease, comprising administering an effective amount of the pharmaceutical composition of the present invention to a subject suffering from or at risk of suffering from a fibrotic disease.

In the preventive or therapeutic method of the present invention, the fibrotic disease may be at least one disease selected from the group consisting of fibrosis, liver cirrhosis, renal failure, myocardial infarction, and cancer.

### [Advantageous Effects of Invention]

According to the present invention, the expression of mRNA encoding P4HA3, which is specifically expressed in myofibroblasts that appear in fibrotic diseases, is inhibited, and therefore, while there are no side effects in patients with diseases other than fibrotic diseases and healthy subjects in whom myofibroblasts are hardly present, it is possible to more strongly suppress the production of fibrosis-related proteins in patients with fibrotic diseases than with conventional therapeutic drugs for fibrotic diseases.

### [Brief Description of the Drawings]

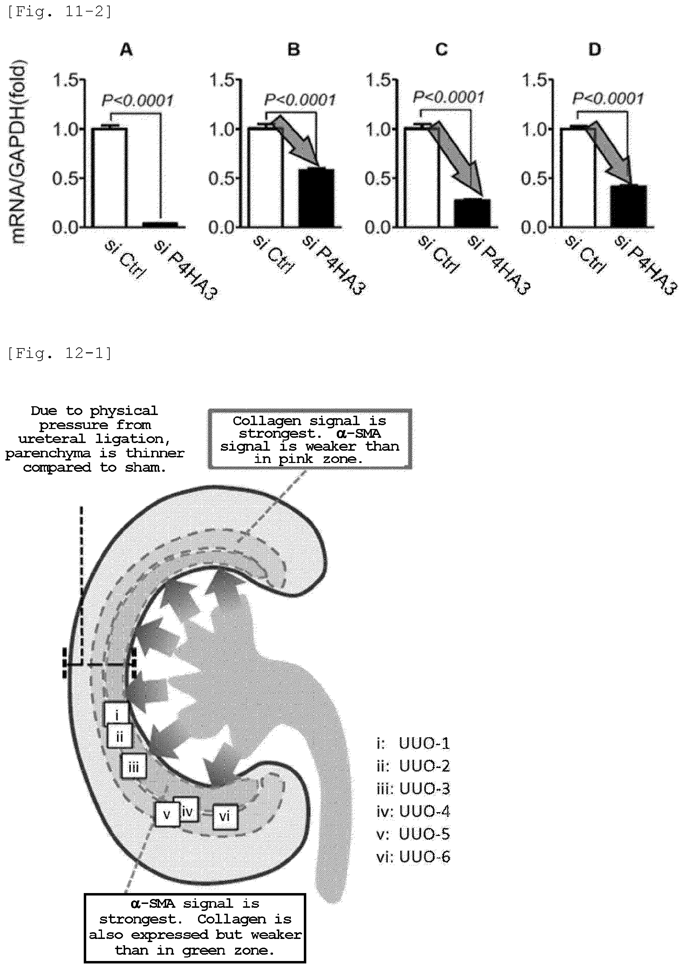

[Fig. 1]
   A graph showing the time course of the relative mRNA expression levels of P4HA3 (A), α-SMA (B), and periostin (C) in the heart after myocardial infarction. ■ indicates the mRNA expression level of each gene measured by real-time RT-PCR in the infarcted area, ▲ indicates the mRNA expression level of each gene measured by real-time RT-PCR in the non-infarcted area, and ● indicates the mRNA expression level of each gene measured by real-time RT-PCR in the sham treatment group. The results are from 3 to 6 experiments performed under the same conditions. The error bars of the expression levels of the mRNA of each gene in the infarcted area 7 days after treatment represent the standard deviation. The vertical axis indicates the relative value of the mRNA expression level of each gene in the heart sample on day 0 of the sham treatment group, where the value obtained by correcting the mRNA expression level of each gene by the GAPDH gene mRNA expression level is taken as 1, and the horizontal axis indicates the number of days after myocardial infarction treatment (unit: days). The number of samples in the infarcted area, non-infarcted area, and sham treatment group were 5-6, 3, and 3-4, respectively. P values were calculated by unpaired two-tailed Student's t-test. ### on the graph indicates that the significance of infarcted area vs. sham treatment group (inf vs. sham) on the same number of days after infarction treatment is p<0.001, and *** on the graph indicates that the significance of infarcted area vs. non-infarcted area (inf vs. rem) on the same number of days after infarction treatment is p<0.001.
[Fig. 2]
   Panel of fluorescent microscopy images showing the results of in situ hybridization of heart sections on day 7 after myocardial infarction. A: A fluorescent microscopy image showing the results of in situ hybridization with periostin and P4HA3 probes, merged with a DAPI-stained fluorescent microscopy image. The scale indicates 20 µm. B, C, and D are enlarged views of the area surrounded by the dashed line in A. B: A fluorescent microscopy image showing the results of in situ hybridization with a periostin probe. C: A fluorescent microscopy image showing the results of in situ hybridization with a P4HA3 probe. D: A fluorescent microscopy image merging the fluorescent microscopy images of B and C. The scale indicates 10 µm.
[Fig. 3]
   t-SNE plots showing the results of single-cell analysis of each cell type in mouse hearts before and after treatment in a myocardial infarction model, created using the data from Farbehi N. et al. (2019). A: Dimensionality reduction plots of cell populations expressing mRNA specific to each cell type. B: Dimensionality reduction plots visualizing cell populations expressing the αSMA (Acta2) gene. C: Dimension reduction plot visualizing the cell population expressing the periostin (Postn) gene. D: Dimension reduction plot visualizing the cell population expressing the P4HA3 gene. Mac indicates macrophage, Endo indicates endothelial cell, Mural indicates smooth muscle cell and pericyte, Fibre indicates fibroblast, and ActFib indicates activated fibroblast cell clusters.
[Fig. 4]
   Bar graph showing the effect of P4HA3 knockdown on the expression of P4HA3 (A), periostin (B), 1α1 collagen (C), and 3α1 collagen (D) genes in myofibroblasts isolated from the infarcted area. si P4HA3 and si Ctrl represent the relative values of mRNA expression levels of each gene measured by real-time RT-PCR in myofibroblasts administered with siRNA against the P4HA3 gene and control gene, respectively. The vertical axis represents the relative value of the mRNA expression level of each gene in the si Ctrl group, where the value obtained by correcting the mRNA expression level of each gene with the mRNA expression level of GAPDH is taken as 1. The number of samples was 5 for each group. P values were calculated using unpaired two-tailed t-test. Error bars indicate standard deviation.
[Fig. 5]
   Bar graphs showing the mRNA expression level of the P4HA3 gene measured by real-time RT-PCR in experimental fibrosis models of the lung (A), liver (B), and kidney (C). Graph A shows the relative expression level of P4HA3 mRNA in lung tissue of the bleomycin-treated group (Bleomycin) or sham-treated group (Saline), graph B shows the relative expression level of P4HA3 mRNA in liver tissue of an ultra-high fat choline-deficient methionine-reduced diet-fed group (NASH) or normal diet-fed group (Ctrl), and graph C shows the relative expression level of P4HA3 mRNA in kidney tissue of the UUO-treated group (UUO) or sham-treated group. The vertical axis of each bar graph shows the relative expression level of P4HA3 mRNA, where the value corrected by the expression level of GAPDH mRNA is taken as 1. P values were calculated by unpaired two-tailed t-test. The number of samples was 5 for the lung (A) and liver (B) fibrosis induction experiments, 3 for the sham treatment group, and 8 for the UUO treatment group for the kidney (C) fibrosis induction experiment. Error bars show standard deviation.
[Fig. 6-1]
   Bar graphs showing the time course of relative mRNA expression levels of P4HA3 (A), α-SMA (B), and 1α1 collagen (C) measured by real-time RT-PCR in liver tissues collected from model mice with liver damage and fibrosis induced by carbon tetrachloride administration at 4 weeks after the start of solvent administration (Vehicle), at 4 weeks after the start of carbon tetrachloride administration (CCl₄), and at 4 weeks after the cessation of carbon tetrachloride administration (Withdraw). The vertical axis of each bar graph represents the relative value of the mRNA expression level of each gene, where the value of the mRNA expression level of each gene in the group 4 weeks after the start of solvent administration (Vehicle) is corrected by the mRNA expression level of GAPDH, with the value set to 1. The number of samples was 5. ** indicates P<0.01, and *** indicates P<0.001. P values were calculated by unpaired two-tailed t-test. Error bars indicate standard deviation.
[Fig. 6-2]
   Bar graph showing the time course of P4HA3 mRNA expression in the liver of diet-induced NASH model mice. Each bar graph (0W, 3W, 6W, 9W, and 12W) shows the time course of relative P4HA3 mRNA expression levels measured by real-time RT-PCR in liver tissue collected from mice before, 3 weeks after, 6 weeks after, 9 weeks after, and 12 weeks after feeding an ultra-high fat, choline-deficient, methionine-reduced diet. The vertical axis of each bar graph shows the relative value of P4HA3 mRNA expression levels at each time point, where the value obtained by correcting the P4HA3 mRNA expression level before feeding by the GAPDH mRNA expression level is set to 1. Results of six experiments performed under the same conditions (five times only after 3 weeks of feeding).
[Fig. 7]
   Bar graph showing the mRNA expression levels of each gene measured by real-time RT-PCR in diet-induced NASH model mice. Each bar graph shows the relative values of P4HA3 mRNA expression level (A), α-smooth muscle actin (α-SMA) mRNA expression level (B), and 1α1 collagen mRNA expression level (C) in liver tissues of NASH-developed mice fed an ultra-high fat, choline-deficient, methionine-reduced diet for 5 weeks and harvested 5 weeks after the diet was stopped (NASH), and in liver tissues of control mice fed a normal diet (Ctrl). The vertical axis shows the relative values of the mRNA expression level of each gene, where the value of the mRNA expression level of each gene in the Ctrl group corrected by the GAPDH gene mRNA expression level is taken as 1. P values were calculated by unpaired two-tailed t-test. The number of samples was 5.
[Fig. 8]
   Bar graph showing the relative mRNA expression levels of Cyp7a1 (A), Cd68 (B), α-SMA (C), and P4HA3 (D) genes measured by real-time RT-PCR for each cell type fraction of liver harvested 4 weeks after the start of carbon tetrachloride administration. The vertical axis represents the relative mRNA expression level of each gene in the hepatic cell fraction, where the mRNA expression level of each gene is corrected by the mRNA expression level of GAPDH, taking as 1. P values were calculated using unpaired two-tailed t-test. The number of samples is 1. Error bars indicate standard deviation.
[Fig. 9]
   Bar graph showing the effect of P4HA3 knockdown on the expression of P4HA3 (A), 1α1 collagen (B), 1α2 collagen (C), 3α1 collagen (D), and elastin (E) genes in liver-derived myofibroblasts from a mouse model of liver fibrosis. The left bar of each bar graph represents the relative expression level of each gene in myofibroblasts administered with siRNA for the control gene (si Ctrl), the center bar represents the first siRNA for the P4HA3 gene (si P4HA3 #1), and the right bar represents the second siRNA for the P4HA3 gene (si P4HA3 #2). The vertical axis represents the relative expression level of each gene's mRNA in the si Ctrl group, where the expression level of each gene is corrected by the expression level of the GAPDH gene mRNA, taking as 1. The results are from four experiments performed under the same conditions. * indicates P<0.05, *** indicates P<0.001. The P value was calculated using the Tukey test. Error bars indicate standard deviation.
[Fig. 10]
   Bar graphs showing the relative mRNA expression levels measured by real-time RT-PCR for P4HA3 (A), α-SMA (B), 1α1 collagen (C), and 3α1 collagen (D) genes in lung tissues from mice with pulmonary fibrosis (bleomycin) and control mice administered saline (saline), collected 7 days after intrabronchial administration of bleomycin. The vertical axis of each graph represents the relative mRNA expression level of each gene, where the value of the mRNA expression level of each gene in the saline group corrected by the mRNA expression level of GAPDH is taken as 1. P values were calculated using unpaired two-tailed t-test. The sample size was 5. Error bars indicate standard deviation.
[Fig. 11-1]
   Bar graph showing the relative values of mRNA expression levels measured by real-time RT-PCR for the genes Cd68 (A), 1α1 collagen (B), and P4HA3 (C) in blood cell fractions and myofibroblast fractions isolated from the lungs of a bleomycin-administered pulmonary fibrosis model mouse. The vertical axis represents the relative value of the mRNA expression level of each gene, where the value obtained by correcting the mRNA expression level of each gene in the hepatic cell fraction by the mRNA expression level of GAPDH is taken as 1.
[Fig. 11-2]
   Bar graph showing the effect of P4HA3 knockdown on the expression of the genes P4HA3 (A), 1α1 collagen (B), 3α1 collagen (C), and 14α1 collagen (D) in myofibroblasts isolated from the lungs of a bleomycin-administered pulmonary fibrosis model mouse and administered si Ctrl or si P4HA3. The vertical axis shows the relative value of the mRNA expression level of each gene in the si Ctrl group, where the value obtained by correcting the mRNA expression level of each gene by the mRNA expression level of GAPDH is set to 1. The results are from five experiments performed under the same conditions. P values were calculated by unpaired two-tailed t-test. Error bars show standard deviation.
[Fig. 12-1]
   Schematic coronal section showing the characteristics of each area of renal lesion in a mouse model of renal fibrosis caused by unilateral ureteral obstruction (UUO). i to vi indicate the positions of kidney tissue sections.
[Fig. 12-2]
   Fluorescence microscopy images showing the results of in situ hybridization using P4HA3 and 1α1 collagen probes, and the results of fluorescent histochemistry using anti-α-SMA antibody for a sham kidney tissue section (sham) corresponding to area i in Fig. 12-1 (all at 20x magnification). The fluorescence microscopy image in the top center shows the results of in situ hybridization using the P4HA3 probe. The top right is a fluorescence microscope image showing the results of fluorescent histochemistry using an anti-α-SMA antibody. The top left is a fluorescence microscope image obtained by merging the fluorescence microscope images shown in the top center and top right with a DAPI-stained fluorescence microscope image. The bottom left is a fluorescence microscope image showing the results of in situ hybridization using a 1α1 collagen probe. The bottom center is a fluorescence microscope image obtained by merging the fluorescence microscope images shown in the top center and top right. The bottom right is a fluorescence microscope image obtained by merging the fluorescence microscope images shown in the top center and bottom left.
[Fig. 12-3]
   Fluorescence microscopy images (all at 20x magnification) showing the results of in situ hybridization using P4HA3 and 1α1 collagen probes, and the results of fluorescent histochemistry using anti-α-SMA antibody, of kidney tissue sections from the UUO-1 region (see region i in Fig. 12-1) on the side where unilateral ureteral obstruction was performed. The top center is a fluorescence microscopy image showing the results of in situ hybridization using the P4HA3 probe. The top right is a fluorescence microscopy image showing the results of fluorescent histochemistry using the anti-α-SMA antibody. The top left is a fluorescence microscopy image merging the fluorescence microscopy images in the top center and top right with a DAPI-stained fluorescence microscopy image. The bottom left is a fluorescence microscopy image showing the results of in situ hybridization using the 1α1 collagen probe. The bottom center is a fluorescence microscopy image merging the fluorescence microscopy images in the top center and top right. The bottom right is a fluorescence microscopy image merging the fluorescence microscopy images in the top center and bottom left.
[Fig. 12-4]
   Fluorescence microscopy images (all at 20x magnification) showing the results of in situ hybridization using P4HA3 and 1α1 collagen probes, and the results of fluorescent histochemistry using anti-α-SMA antibody, of kidney tissue sections from the UUO-2 region (see region ii in Fig. 12-1) on the side where unilateral ureteral obstruction was performed. The top center is a fluorescence microscopy image showing the results of in situ hybridization using the P4HA3 probe. The top right is a fluorescence microscopy image showing the results of fluorescent histochemistry using the anti-α-SMA antibody. The top left is a fluorescence microscopy image that merges the fluorescence microscopy images in the top center and top right with a DAPI-stained fluorescence microscopy image. The bottom left is a fluorescence microscopy image showing the results of in situ hybridization using the 1α1 collagen probe. The bottom center is a fluorescence microscopy image that merges the fluorescence microscopy images in the top center and top right. The bottom right is a fluorescence microscopy image that merges the fluorescence microscopy images in the top center and bottom left.
[Fig. 12-5]
   Fluorescence microscopy images (all at 20x magnification) showing the results of in situ hybridization using P4HA3 and 1α1 collagen probes, and the results of fluorescent immunohistochemistry using anti-α-SMA antibody, of kidney tissue sections from the UUO-3 region (see region iii in Fig. 12-1) on the side where unilateral ureteral obstruction was performed. The top center is a fluorescence microscopy image showing the results of in situ hybridization using the P4HA3 probe. The top right is a fluorescence microscopy image showing the results of fluorescent histochemistry using the anti-α-SMA antibody. The top left is a fluorescence microscopy image that merges the fluorescence microscopy images in the top center and top right with a DAPI-stained fluorescence microscopy image. The bottom left is a fluorescence microscopy image showing the results of in situ hybridization using the 1α1 collagen probe. The bottom center is a fluorescence microscopy image that merges the fluorescence microscopy images in the top center and top right. The bottom right is a fluorescence microscopy image that merges the fluorescence microscopy images in the top center and bottom left.
[Fig. 12-6]
   Fluorescence microscopy images (all at 20x magnification) showing the results of in situ hybridization using P4HA3 and 1α1 collagen probes, and the results of fluorescent histochemistry using anti-α-SMA antibody, of kidney tissue sections from the UUO-5 region (see region v in Fig. 12-1) on the side where unilateral ureteral obstruction was performed. The top center is a fluorescence microscopy image showing the results of in situ hybridization using the P4HA3 probe. The top right is a fluorescence microscopy image showing the results of fluorescent histochemistry using the anti-α-SMA antibody. The top left is a fluorescence microscopy image that merges the fluorescence microscopy images in the top center and top right with a DAPI-stained fluorescence microscopy image. The bottom left is a fluorescence microscopy image showing the results of in situ hybridization using the 1α1 collagen probe. The bottom center is a fluorescence microscopy image that merges the fluorescence microscopy images in the top center and top right. The bottom right is a fluorescence microscopy image that merges the fluorescence microscopy images in the top center and bottom left.
[Fig. 13]
   Schematic diagram showing the relationship between changes in the expression levels of the P4HA3 gene and fibrosis-related genes (1α1 collagen and α-SMA) during the progression of fibrosis.
[Fig. 14]
   Western blotting diagram showing the expression of P4HA3 protein in P4HA3 gene-deficient mice (P4HA3-KO) and wild-type mice (WT). The upper figure shows the result of polyacrylamide gel electrophoresis separation of total proteins of hepatic myofibroblasts, detected with an antibody against the P4HA3 protein, and the lower figure shows the result of detection of the said separation with an antibody against the GAPDH protein.
[Fig. 15-1]
   Line graph showing the time course of body weight change rate when P4HA3 gene-deficient mice (P4HA3-KO) and wild-type mice (WT) were fed an ultra-high fat, choline-deficient, methionine-reduced diet. From the top, the top line graph represents wild-type mice fed a normal diet (WT Ctrl), below that represents P4HA3 gene-deficient mice fed a normal diet (P4HA3-KO Ctrl), next represents wild-type mice fed an ultra-high fat, choline-deficient, methionine-reduced diet (WT NASH), and the bottom line graph represents P4HA3 gene-deficient mice fed an ultra-high fat, choline-deficient, methionine-reduced diet (P4HA3-KO NASH).
[Fig. 15-2]
   Bar graphs showing the relative percentage of liver weight to body weight after feeding an ultra-high fat, choline-deficient, methionine-reduced diet or a normal diet for 10 weeks. The bar graphs show, from the far left, the percentage of liver weight relative to body weight for wild-type mice fed a normal diet (WT Ctrl), wild-type mice fed an ultra-high fat, choline-deficient, methionine-reduced diet (WT NASH), P4HA3 gene-deficient mice fed a normal diet (P4HA3-KO Ctrl), and P4HA3 gene-deficient mice fed an ultra-high fat, choline-deficient, methionine-reduced diet (P4HA3-KO NASH) on the far right. The vertical axis shows the relative liver weight/body weight of each mouse group when the liver weight/body weight of wild-type mice fed a normal diet is taken as 100%. The number of mice in each group ranged from 8 to 10. *** indicates P<0.001. P values were calculated using Tukey's test. Error bars indicate standard deviation.
[Fig. 16]
   Bar graphs comparing the relative expression levels of 1α1 collagen (A), 1α2 collagen (B), 3α1 collagen (C), and elastin (D) in P4HA3 gene-deficient mice and wild-type mice after 10 weeks of feeding a high-fat, choline-deficient, methionine-reduced diet or a normal diet. Each bar graph represents, from the left, the mRNA expression level of each gene in wild-type mice fed a normal diet (WT Ctrl), wild-type mice fed an ultra-high fat, choline-deficient, methionine-reduced diet (WT NASH), P4HA3 gene-deficient mice fed a normal diet (P4HA3-KO Ctrl), and, on the far right, P4HA3 gene-deficient mice fed an ultra-high fat, choline-deficient, methionine-reduced diet (P4HA3-KO NASH). The vertical axis shows the relative value of the mRNA expression level of each gene, where the value of the mRNA expression level of each gene in WT Ctrl corrected by the mRNA expression level of GAPDH is set to 1. The number of mice in each group ranged from 8 to 10. *** indicates P<0.001. P values were calculated using Tukey's test. Error bars indicate standard deviation.
[Fig. 17]
   The left panel shows Picrosirius Red stained optical microscope images of liver sections from P4HA3 gene-deficient mice (P4HA3-KO) or wild-type mice (WT) fed an ultra-high fat, choline-deficient, methionine-reduced diet (NASH) or normal diet (Ctrl). The scale indicates 1 mm. The right panel shows the results of evaluation of these microscopic images by histopathological analysis. Each bar graph shows, from the left, the percentage of collagen accumulation area in the liver of a wild-type mouse fed a normal diet (WT Ctrl), a wild-type mouse fed an ultra-high fat, choline-deficient, methionine-reduced diet (WT NASH), a P4HA3 gene-deficient mouse fed a normal diet (P4HA3-KO Ctrl), and, on the far right, a P4HA3 gene-deficient mouse fed an ultra-high fat, choline-deficient, methionine-reduced diet (P4HA3-KO NASH). The vertical axis of the graph shows the percentage of collagen accumulation area. There were six mice in each group. *** indicates P<0.001. P values were calculated using Tukey's test. Error bars show standard deviation.
[Fig. 18]
   A bar graph showing the results of an analysis of liver function markers AST (left) or ALT (right) in the serum of P4HA3 gene-deficient mice (P4HA3-KO) or wild-type mice (WT) fed an ultra-high fat, choline-deficient, methionine-reduced diet (NASH) or a normal diet (Ctrl). Each bar graph represents the enzyme activity of AST or ALT in, from the left, wild-type mice fed a normal diet (WT Ctrl), wild-type mice fed an ultra-high fat, choline-deficient, methionine-reduced diet (WT NASH), P4HA3 gene-deficient mice fed a normal diet (P4HA3-KO Ctrl), and, on the far right, P4HA3 gene-deficient mice fed an ultra-high fat, choline-deficient, methionine-reduced diet (P4HA3-KO NASH). The vertical axis of the graph represents the enzyme activity of AST or ALT (unit: IU/L). The number of mice in each group ranged from 5 to 6. The vertical axis represents the enzyme activity of AST or ALT. * indicates P<0.05, ** indicates P<0.01. P values were calculated using Tukey's test. Error bars indicate standard deviation.
[Fig. 19]
   Bar graph showing the relative values of mRNA expression levels of P4HA3 (A) and Hsp47 (B) genes measured by real-time T-PCR in the hepatocyte fraction, blood cell fraction, and myofibroblast fraction of the liver of a mouse model of liver damage and fibrosis caused by carbon tetrachloride administration. The vertical axis represents the relative values of mRNA expression levels in each cell fraction, where the value obtained by correcting the mRNA expression level of each gene in the hepatocyte fraction by the mRNA expression level of GAPDH is taken as 1.
[Fig. 20]
   Graph A is a bar graph showing the relative expression level of the Hsp47 gene in myofibroblasts derived from fibrotic livers induced by carbon tetrachloride which were administered with si Hsp47 or si Ctrl. Graph B is a bar graph showing the relative expression level of the P4HA3 gene in myofibroblasts administered with si P4HA3 or si Ctrl. Graphs C to F are bar graphs showing the relative expression levels of 1α1 collagen (C), 3α1 collagen (D), 8α1 collagen (E), and 14α1 collagen (F) genes in myofibroblasts administered with si Ctrl, si Hsp47, and si P4HA3, respectively. The vertical axis shows the relative expression level of the mRNA of each gene in the si Ctrl group, where the value obtained by correcting the mRNA expression level of each gene by the mRNA expression level of GAPDH is taken as 1. The number of samples for each group is 4. ** indicates P<0.01, *** indicates P<0.001, and n.s. indicates no significant difference. P values were calculated by Tukey test. Error bars indicate standard deviation.
[Fig. 21-1]
   Graphs A to D are bar graphs showing the relative mRNA expression levels of Hsp47 (A), P4HA3 (B), 1α1 collagen (C), or 1α2 collagen (D) in myofibroblasts derived from damage and fibrotic livers administered with siHsp 47 and/or si P4HA3. The vertical axis shows the relative mRNA expression level of each gene in the si Ctrl group, where the mRNA expression level of each gene is corrected by the mRNA expression level of GAPDH, and the value is set to 1. The number of samples for each group is 4. *** indicates P<0.001, and n.s. indicates no significant difference. P values were calculated by Tukey test. Error bars indicate standard deviation.
[Fig. 21-2]
   Graphs A to D are bar graphs showing the relative expression levels of 3α1 collagen (A), 8α1 collagen (B), 14α1 collagen (C), or elastin (D) genes in myofibroblasts derived from damaged fibrotic livers administered si Hsp47 and/or si P4HA3. The vertical axis shows the relative expression level of each gene mRNA, where the value of the mRNA expression level of each gene in the si Ctrl group corrected by the mRNA expression level of GAPDH is taken as 1. The number of samples for each group is 5. * indicates P<0.05, ** indicates P<0.01, *** indicates P<0.001, and n.s indicates no significant difference. P values were calculated by Tukey's test. Error bars indicate standard deviation.
[Fig. 22-1]
   Kaplan-Meier curves showing the relationship between the level of P4HA3 gene expression and the prognosis of bladder cancer (A), head and neck squamous cell carcinoma (B), cervical squamous cell carcinoma (C), and hepatocellular carcinoma (D). Cases with high P4HA3 gene expression are shown in black, and cases with low P4HA3 gene expression are shown in grey. All were created using Kaplan-Meier Plotter.
[Fig. 22-2]
   Kaplan-Meier curves showing the relationship between the level of P4HA3 gene expression and the prognosis of pancreatic ductal carcinoma (E), breast cancer (F), gastric adenocarcinoma (G), and lung squamous cell carcinoma (H). Cases with high P4HA3 gene expression are shown in black, and cases with low P4HA3 gene expression are shown in grey. All were created using Kaplan-Meier Plotter.
[Fig. 22-3]
   Kaplan-Meier curves showing the relationship between the level of P4HA3 gene expression and the prognosis of rectal adenocarcinoma (I) and ovarian cancer (J). Cases with high expression of the P4HA3 gene are shown in black, and cases with low expression of the P4HA3 gene are shown in grey. All were created using Kaplan-Meier Plotter.
[Fig. 23]
   The top graph shows the time course of the relative expression levels of different isoforms of P4HA3 mRNA, P4HA3, P4HA1, and P4HA2, in the heart after myocardial infarction. ■ indicates the mRNA expression level of each gene in the infarcted area, ▲ indicates the mRNA expression level of each gene in the non-infarcted area, and ● indicates the mRNA expression level of each gene in the sham treatment group, measured by real-time RT-PCR. The vertical axis represents the relative value of the mRNA expression level of each gene, where the value of the mRNA expression level of each gene on the day of myocardial infarction treatment (0) corrected by the mRNA expression level of GAPDH is taken as 1, and the horizontal axis represents the number of days after myocardial infarction treatment (unit: days). The lower part is a t-SNE plot created using the data from Farbehi N. et al. (2019) mentioned above, which is a dimension reduction plot visualizing cell populations expressing P4HA3 and P4HA1 and P4HA2, which are isoforms different from P4HA3.
[Fig. 24-1]
   The top bar graph shows the relative mRNA expression levels of 1α1 collagen gene (Col1α1) and the different isoforms of P4HA3 mRNA, P4HA3, P4HA1, and P4HA2, in cardiac tissue from myocardial infarction induced by transverse aortic coarctation (TAC) or cardiac tissue from sham-treated control mice (Sham). The bottom bar graph shows the relative mRNA expression levels of 1α1 collagen (Col1α1) and the different isoforms of P4HA, P4HA3, P4HA1, and P4HA2, in lung tissue from bleomycin-administered pulmonary fibrosis model mice (BLM) or lung tissue from saline-administered control mice (Saline). The vertical axis shows the relative value of the mRNA expression level of each gene, where the value of the mRNA expression level of each gene in the sham or saline group corrected by the mRNA expression level of GAPDH is set to 1. * indicates P<0.05, *** indicates P<0.001, and n.s. indicates no significant difference. P values were calculated using Student's t-test. Error bars indicate standard deviation. There were 5 mice in each mouse group.
[Fig. 24-2]
   The top row is a bar graph showing the relative values of the mRNA expression levels of 1α1 collagen gene (Col1α1) and the different P4HA isoforms P4HA3, P4HA1, and P4HA2 in liver tissue from mice that developed NASH by feeding an ultra-high fat, choline-deficient, methionine-reduced diet (NASH) or in liver tissue from control mice fed a normal diet (Ctrl). The lower part is a bar graph showing the relative values of the mRNA expression levels of 1α1 collagen mRNA (Col1α1) and different P4HA isoforms P4HA3, P4HA1, and P4HA2 in the renal lesions of a mouse model of renal fibrosis caused by unilateral ureteral obstruction (UUO) or in the renal tissues of a sham-treated control mouse. The vertical axis shows the relative value of the mRNA expression level of each gene, where the value obtained by correcting the mRNA expression level of each gene in the Ctrl or Sham group by the mRNA expression level of GAPDH is taken as 1. *** indicates P<0.001. The P value was calculated by Student's t-test. Error bars indicate standard deviation. There are 5 mice in each group.
[Fig. 25]
   A bar graph showing the effects of si Ctrl, si P4HA1, si P4HA2, and si P4HA3 on gene expression of fibrosis-related factors (P4HA1, P4HA2, P4HA3, 1α1 collagen (Col1α1), 1α2 collagen (Col1α2), 3α1 collagen (Col3α1), and elastin (Eln)) in myofibroblasts isolated from liver tissue of a mouse model with liver damage and fibrosis caused by carbon tetrachloride administration. The vertical axis shows the relative value of the mRNA expression level of each gene, where the value obtained by correcting the mRNA expression level of each gene in the si Ctrl group by the mRNA expression level of GAPDH is taken as 1. The number of samples for each group was 4. Comparisons between groups were calculated using one-way analysis of variance followed by Tukey's range test. * indicates P<0.05, ** indicates P<0.01, *** indicates P<0.001, and n.s. indicates no significant difference. Error bars indicate standard deviation.
[Fig. 26]

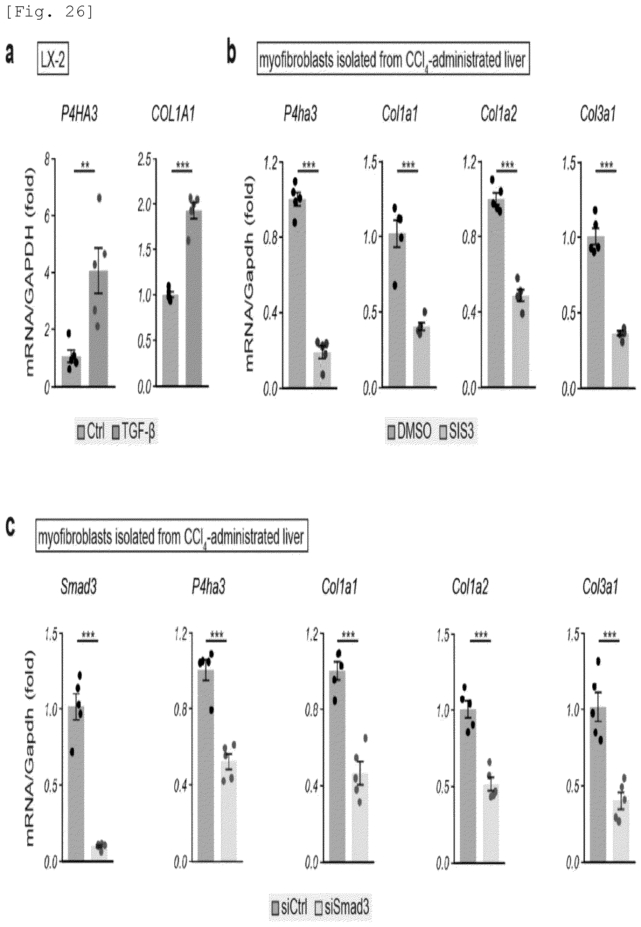

   Fig. 26a is a bar graph showing the mRNA expression levels of P4HA3 or 1α1 collagen (Col1α1) in LX-2 treated for 12 hr with PBS or PBS containing TGFβ (5 ng/mL). The vertical axis shows the relative value of the mRNA expression level of each gene in the Ctrl group, where the value obtained by correcting the mRNA expression level of each gene by the mRNA expression level of GAPDH is taken as 1. There were three samples for each group. Comparisons between groups were calculated by one-way analysis of variance followed by Tukey's range test. * indicates P<0.05, ** indicates P<0.01, *** indicates P<0.001, and n.s. indicates no significant difference. Error bars indicate standard deviation. Fig. 26b is a bar graph showing the mRNA expression levels of P4HA3, 1α1 collagen (Col1α1), 1α2 collagen (Col1α2), and 3α1 collagen (Col3α1) in myofibroblasts isolated from liver tissue of a mouse model of liver damage and fibrosis caused by carbon tetrachloride administration and treated with SIS3, an inhibitor of the TGFβ/SMAD pathway. The vertical axis shows the relative value of the mRNA expression level of each gene, where the value corrected by the mRNA expression level of each gene in the si Ctrl group by the mRNA expression level of GAPDH is set to 1. The number of samples for each group was 5. Comparisons between groups were calculated by one-way analysis of variance followed by Tukey's range test. * indicates P<0.05, ** indicates P<0.01, *** indicates P<0.001, and n.s indicates no significant difference. Fig. 26c is a bar graph showing the mRNA expression levels of SMAD3, P4HA3, 1α1 collagen (Col1α1), 1α2 collagen (Col1α2), and 3α1 collagen (Col3α1) in myofibroblasts isolated from liver tissue of a mouse model of liver damage and liver fibrosis caused by carbon tetrachloride administration and transfected with siRNA against SMAD3 (si Smad3) or si Ctrl. The vertical axis shows the relative value of the mRNA expression level of each gene in the si Ctrl group, where the value obtained by correcting the mRNA expression level of each gene by the mRNA expression level of GAPDH is taken as 1. The number of samples for each group was 5. Comparisons between groups were calculated by one-way analysis of variance followed by Tukey's range test. * indicates P<0.05, ** indicates P<0.01, *** indicates P<0.001, and n.s indicates no significant difference.

### [Description of Embodiments]

### 1. Definition

In the present invention, fibrotic diseases refer to all diseases characterized by physical hardening of organs caused by the overproduction of fibrous proteins. Fibrotic diseases include, but are not limited to, myocardial infarction, cirrhosis, renal failure, fibrosis of the lungs and other organs, and some cancers. The elastic modulus of a normal liver is about 5 kPa, but increases to about 8-75 kPa in the lesions of fatty liver or cirrhosis. The elastic modulus of a normal heart is about 10 kPa, but increases to about 35-70 kPa in the lesions of myocardial infarction. In any fibrotic disease, the physical hardening of organs leads to a decrease in the function of each organ. The physical hardening of organs is caused by the excessive accumulation of fibrous proteins, which are extracellular matrix components such as collagen and elastin, in the organs. Therefore, the fibrous proteins are called fibrosis-related factors.

In recent years, it has been revealed that in myocardial infarction, liver cirrhosis, and renal failure, as well as fibrosis in the lungs, skin, and other organs, the fibrosis-related factors are overproduced by a specific cell type called myofibroblasts (also called activated stellate cells in the liver). Myofibroblasts are rarely present in normal organs. Triggered by inflammatory reactions, ischemic reactions, mechanical stimuli, etc., myofibroblasts differentiate from fibroblasts, pericytes, endothelial cells, epithelial cells, and bone marrow-derived cells that are always present in normal tissues. Myofibroblasts have contractile ability, and α-smooth muscle actin (hereinafter also referred to as α-SMA or ACTA2) is a marker specific to myofibroblasts. It is also known that cancer lesions can sometimes be physically hardened compared to the surrounding normal tissue. Even in physically hardened cancer lesions, the overproduction of fibrosis-related factors by myofibroblasts is involved. Traditionally, fibrotic diseases have been classified separately according to the cause of physical hardening, such as inflammation, ischemia, malignant neoplasms, etc., such as liver cirrhosis, myocardial infarction, and pulmonary fibrosis. It has been revealed in recent years that even if the organs and causes of physical hardening are different, they share a common pathology of physical hardening due to the overproduction of fibrosis-related factors by myofibroblasts. Therefore, in the present specification, all diseases characterized by physical hardening of organs caused by the overproduction of fibrous proteins are called fibrotic diseases. Fibrotic diseases include, but are not limited to, myocardial infarction, liver cirrhosis, and renal failure, fibrosis of the lungs, skin, and other organs, and cancer accompanied by physical hardening of lesions.

### 2. Search for genes specifically expressed in myofibroblasts

In the present invention, the search for genes specifically expressed in myofibroblasts was performed by DNA microarray analysis and single-cell expression profiling, both of which are well known to those skilled in the art (see Luecken, M.D. & Theis, F.J. (2019) Molecular Systems Biology 15: e8746, Farbehi N. et al, (2019) eLife 2019; 8:e43882.). In the single-cell analysis of fibrotic diseases of the present invention, cells or cell nuclei of an organ or tissue are dissociated, and each isolated cell is physically isolated, for example, by encapsulating it in a droplet or sorting it into a well of a microwell plate. A gel bead is placed in each droplet or well in advance. A reverse transcription primer having a different DNA barcode, i.e., a unique molecular identifier (UMI), is immobilized on each gel bead. When cDNA synthesis reaction, cDNA library creation, and cDNA sequencing are performed in each droplet or well, cDNA sequence data containing UMI-derived sequences at the 3' end of mRNA derived from individual cells is obtained. The obtained cDNA sequence data is subjected to computer analysis. Single cell analysis often uses a single cell gene expression analysis system sold by 10x Genomics and a next-generation sequencer sold by Illumina and others, but other reagents, kits, equipment, and/or software may also be used.

In computer analysis of cDNA sequence data for single cell analysis, first, quality control, such as distinguishing data derived from intact cells from data derived from cells whose cell membranes have been damaged and some of the mRNA has leaked, and normalization are performed. Then, the similarity of gene expression states of cells is intuitively displayed by dimensionality reduction using methods such as t-distributed Stochastic Neighbor Embedding (tSNE) (see Kobak, D. & Berens, P. (2019) Nat Commun., 10(1):5416).

### 3. Nucleic acid contained in the pharmaceutical composition of the present invention

One embodiment of the present invention is a pharmaceutical composition for treating or preventing a fibrotic disease. The pharmaceutical composition contains a nucleic acid that inhibits the expression of mRNA encoding the α3 subunit of prolyl 4-hydroxylase (P4HA3). The mRNA encoding the α3 subunit of prolyl 4-hydroxylase (P4HA3) whose expression is inhibited by the nucleic acid contained in the pharmaceutical composition of the present invention includes, but is not limited to, humans and other primates, and mice and other rodents. In the present specification, the term "knockdown" refers to the inhibition of mRNA expression of a gene by a nucleic acid that inhibits the expression of mRNA encoding the gene. For example, the term "P4HA3 knockdown" refers to the inhibition of mRNA expression of P4HA3 by a nucleic acid that inhibits the expression of mRNA encoding P4HA3.

Prolyl 4-hydroxylase is an enzyme that catalyzes the reaction of converting proline residues in collagen and elastin into hydroxyproline through post-translational hydroxylation modification. Hydroxyproline stabilizes the triple-stranded helical structure of collagen. Prolyl 4-hydroxylase is a tetrameric protein consisting of two α subunits and two β subunits, and the α subunit of prolyl 4-hydroxylase has three isoforms: the α1 subunit (P4HA1), the α2 subunit (P4HA2), and the α3 subunit (P4HA3). The β subunit is a multifunctional protein that is known to function as a protein disulfide isomerase in a monomer. As demonstrated in the following examples, in fibrotic disease models of the heart, lung, liver, and kidney, the expression of P4HA3 was increased in the treatment group compared to the sham treatment group, but there was no significant difference in the expression of P4HA1 and P4HA2 between the sham treatment group and the treatment group, or even if there was a significant difference, the difference in expression between the sham treatment group and the treatment group was not as large as that of P4HA3.

As explained in the examples of the present specification, the pharmaceutical composition of the present invention suppresses the mRNA expression of fibrosis-related factors by suppressing the mRNA expression of P4HA3 produced by myofibroblasts. Therefore, by administering the pharmaceutical composition of the present invention to not only patients with symptoms of fibrotic disease, but also subjects with myocardial infarction and inflammatory diseases of the liver, lungs, kidneys, and other conditions that cause fibrotic disease, but who have not yet developed fibrotic disease, it is possible to prevent fibrotic disease by suppressing the mRNA expression of fibrosis-related factors by myofibroblasts.

The nucleotide sequence of DNA encoding human P4HA3 mRNA is registered in the NCBI Reference Sequence as, for example, NM_182904.4, NM_182904.5, NM_001288748.1, NM_001288748.2, XR_001747836.1, and XR_001747837.1. The nucleotide sequence of DNA encoding mouse P4HA3 mRNA is registered in the NCBI Reference Sequence as, for example, NM_177161.4. Specific embodiments of the nucleic acid contained in the pharmaceutical composition of the present invention include, but are not limited to, the following (a) to (d).

### (a) siRNA inhibiting the expression of mRNA encoding P4HA3 or precursor thereof

An embodiment of the nucleic acid of the present invention that inhibits the expression of mRNA encoding P4HA3 is a double-stranded RNA consisting of an oligoRNA complementary to the mRNA encoding P4HA3 and its complementary strand, i.e., siRNA. siRNA can be designed based on the cDNA sequence information of the target gene, for example, according to the rules proposed by Elbashir et al. (Genes Dev., 15, 188-200 (2001)). In addition, short hairpin RNA (shRNA), which is a precursor of siRNA, can be designed by appropriately selecting any linker sequence (e.g., about 5-25 bases) that can form a loop structure and linking the sense strand and antisense strand of siRNA via the linker sequence.

The sequences of siRNA and/or shRNA can be searched using search software provided free of charge on various websites. Examples of such sites include, but are not limited to, the siDESIGN Center provided by Dharmacon (http://dharmacon.horizondiscovery.com/jp/design-center/?rdr=true&LangType=1041&pageid=17179928204) and the siRNA Target Finder provided by GenScript (https://www.genscript.com/tools/sirna-target-finder).

For example, a candidate siRNA that inhibits the expression of human P4HA3 mRNA contains a nucleotide sequence complementary to a sequence consisting of at least 15 consecutive nucleotides of the full-length sequence of the nucleotide sequence corresponding to human P4HA3 mRNA (variant 1: NM_001288748, SEQ ID NO: 9, full-length 2255 nucleotides, or variant 2: NM_001288748, SEQ ID NO: 10, full-length 2248 nucleotides). Similarly, a candidate siRNA that inhibits the expression of mouse P4HA3 mRNA contains a nucleotide sequence complementary to a sequence consisting of at least 15 consecutive nucleotides of the full-length sequence of the nucleotide sequence corresponding to mouse P4HA3 mRNA (NM_177161.4, SEQ ID NO: 11, full-length 2361 nucleotides).

The siRNA of the present invention that inhibits the expression of P4HA3 mRNA includes a double-stranded RNA containing a nucleotide sequence complementary to a sequence consisting of at least 15 consecutive nucleotides of the full-length DNA sequence listed in SEQ ID NO: 9 or 10. The siRNA of the present invention includes, but is not limited to, a double-stranded RNA containing an RNA sequence listed in SEQ ID NO: 5 and 6, or a double-stranded RNA containing an RNA sequence listed in SEQ ID NO: 7 and 8, or a double-stranded RNA consisting of an RNA sequence listed in SEQ ID NO: 1 and 2, or a double-stranded RNA consisting of an RNA sequence listed in SEQ ID NO: 3 and 4. The two RNAs constituting the siRNA of the present invention may be composed of nucleotide sequences that are completely complementary to each other, but may also contain one or several non-complementary nucleotides at the 5' and/or 3' ends of the completely complementary nucleotide sequences.

The sequences listed in the sequence listing attached to the present specification are as follows.
SEQ ID NO: 1 Sense strand of siRNA #1 (si P4HA3 #1) against mouse P4HA3
SEQ ID NO: 2 Antisense strand of mouse si P4HA3 #1
SEQ ID NO: 3 Sense strand of siRNA #2 (si P4HA3 #2) against mouse P4HA3
SEQ ID NO: 4 Antisense strand of mouse si P4HA3 #2
SEQ ID NO: 5 Nucleotide sequence of SEQ ID NO: 1, which is completely complementary to the nucleotide sequence of SEQ ID NO: 2
SEQ ID NO: 6 Nucleotide sequence of SEQ ID NO: 2, which is completely complementary to the nucleotide sequence of SEQ ID NO: 1
SEQ ID NO: 7 Nucleotide sequence of SEQ ID NO: 3, which is completely complementary to the nucleotide sequence of SEQ ID NO: 4
SEQ ID NO: 8 Nucleotide sequence of SEQ ID NO: 4, which is completely complementary to the nucleotide sequence of SEQ ID NO: 3
SEQ ID NO: 9 Nucleotide sequence variant 1 corresponding to human P4HA3 mRNA (NM_182904.5, total length 2255 nucleotides)
SEQ ID NO: 10 Nucleotide sequence variant 2 corresponding to human P4HA3 mRNA (NM_001288748, full length 2248 nucleotides)
SEQ ID NO: 11 Nucleotide sequence corresponding to mouse P4HA3 mRNA (NM_177161.4, full length 2361 nucleotides)
SEQ ID NO: 12 Sense strand of siRNA (si Hsp47) for mouse Hsp47
SEQ ID NO: 13 Antisense strand of siRNA (si Hsp47) for mouse Hsp47
SEQ ID NO: 14 Sense strand of siRNA (si SMAD3) for mouse SMAD3
SEQ ID NO: 15 Antisense strand of siRNA (si SMAD3) for mouse SMAD3

The nucleotide molecules constituting the siRNA and/or shRNA may contain only natural ribonucleotides, but may also contain deoxyribonucleotides or natural or non-natural modified nucleotides. In order to reduce off-target effects or to improve stability (chemical and/or enzymatic) or specific activity (affinity with RNA), various chemical modifications known per se may be included.

As siRNAs against mRNA encoding human P4HA3, for example, siRNAs targeting the first and second exons, the second exon, or the ninth exon of the human P4HA3 gene are commercially available from Thermo Fisher Scientific Inc. Similarly, as siRNAs against mRNA encoding mouse P4HA3, for example, siRNAs targeting the first and second exons, the second exon, the third exon, the fourth exon, the fourth and fifth exons, the sixth exon, the seventh exon, the eighth and ninth exons, or the thirteenth exon of the mouse P4HA3 gene are commercially available from Thermo Fisher Scientific Inc. Pre-designed or newly designed siRNAs for mRNA encoding human P4HA3 can also be obtained from BIONEER CORPORATION (Daejeon, Korea), which is represented by Cosmo Bio Co., Ltd., and others.

The siRNA can be prepared by synthesizing the sense and antisense strands of the target sequence on the mRNA using an automatic DNA/RNA synthesizer, denaturing them in an appropriate annealing buffer at about 90 to about 95°C for about 1 min, and then annealing them at about 30 to about 70°C for about 1 to about 8 hr. Alternatively, it can be prepared by synthesizing shRNA, which is a precursor of siRNA, and cleaving it with a dicer.

In the present specification, a nucleic acid designed to generate siRNA and/or shRNA that inhibits the expression of mRNA encoding P4HA3 in vivo is also defined as being included in the nucleic acid that inhibits the expression of the mRNA. Examples of such nucleic acids include expression vectors constructed to express the above-mentioned shRNA or siRNA. Although a pol III promoter (e.g., CMV immediate early promoter) can also be used as a promoter, a pol IIII promoter is generally used to ensure accurate transcription of short RNA. Examples of pol IIII promoters include mouse and human U6-snRNA promoters, human H1-RNase P RNA promoter, and human valine-tRNA promoter. In addition, a sequence of four or more consecutive Ts is used as a transcription termination signal.

### (b) Antisense nucleic acid against mRNA encoding P4HA3

An antisense nucleic acid against mRNA encoding P4HA3 is a nucleic acid containing a nucleotide sequence complementary to the nucleotide sequence of the mRNA or a part thereof, and has the function of inhibiting protein synthesis by binding to the target mRNA by forming a specific and stable double strand. The antisense nucleic acid may be DNA or RNA, or may be a DNA/RNA chimera. When the antisense nucleic acid is DNA, the RNA:DNA hybrid formed by the target RNA and the antisense DNA can be recognized by endogenous RNase H to cause selective degradation of the target RNA. The target region of the antisense nucleic acid is not particularly limited in length as long as the translation into protein is inhibited as a result of hybridization with the antisense nucleic acid, and may be the entire sequence or a partial sequence of the mRNA encoding the protein, with a short one being about 10 bases and a long one being the entire sequence of the mRNA or initial transcription product. In addition, antisense nucleic acids may not only hybridize with target mRNA or initial transcription products to inhibit translation into proteins, but may also bind to these genes, which are double-stranded DNA, to form triplexes and inhibit transcription into RNA (antigenes).

The nucleotide molecules constituting the antisense nucleic acid may also be modified in the same manner as the above-mentioned siRNAs, etc., in order to improve stability, specific activity, etc.

Antisense oligonucleotides can be prepared by determining the target sequence of mRNA or initial transcription products based on the cDNA sequence or genomic DNA sequence of the target gene, and synthesizing a complementary sequence to this using a commercially available DNA/RNA automatic synthesizer.

In the present specification, nucleic acids designed to generate antisense RNA against mRNA encoding P4HA3 in vivo are also defined as being included in the antisense nucleic acid against said mRNA. Examples of such nucleic acids include expression vectors constructed to express the above-mentioned antisense RNA. As the promoter, a pol III promoter or a pol IIII promoter can be appropriately selected and used depending on the length of the antisense RNA to be transcribed.

### (c) Ribozyme Nucleic Acid against P4HA3-Encoding mRNA

Ribozyme nucleic acid capable of specifically cleaving the P4HA3-encoding mRNA within the coding region can also be used as a nucleic acid that suppresses the expression of the P4HA3-encoding mRNA. In the narrow sense, "ribozyme" refers to RNA having an enzyme activity that cleaves nucleic acids, but in the present specification, it is used as a concept that includes DNA as long as it has sequence-specific nucleic acid cleavage activity. The most versatile ribozyme nucleic acid is the self-splicing RNA found in infectious RNA such as viroids and virusoids, and hammerhead and hairpin types are known. When a ribozyme is used in the form of an expression vector containing DNA encoding it, it can also be made into a hybrid ribozyme by further linking a sequence obtained by modifying tRNA in order to promote the transfer of the transcript to the cytoplasm.

### (d) miRNA for mRNA encoding P4HA3

MicroRNA (miRNA) targeting mRNA encoding P4HA3 can also be used as a nucleic acid that inhibits the expression of P4HA3 after transcription by binding complementarily to the mRNA encoding P4HA3 to inhibit the translation of the mRNA or by degrading the mRNA. The sequence of miRNA targeting mRNA encoding P4HA3 can be searched using search software provided free of charge on various websites. Examples of such sites include, but are not limited to, TargetScan (http://www.targetscan.org/vert_80/) published by the Whitehead Institute in the United States. Examples of miRNAs that target mRNA encoding P4HA3 include hsa-miR-335-5p (MIRT018485), hsa-miR-124-3p (MIRT022868), mmu-miR-466i-5p (MIRT586397), mmu-miR-466k (MIRT586399), and mmu-miR-466d-5p (MIRT586400).

The nucleic acid that inhibits the expression of mRNA encoding P4HA3 contained in the pharmaceutical composition of the present invention may contain at least one of the above types of nucleic acid (a) to (d), and may contain two or more types of nucleic acid of different molecular species of the same type, or may contain two or more different types of nucleic acid.

### 4. Nucleic acid delivery carrier contained in the pharmaceutical composition of the present invention

As long as the nucleic acid delivery carrier contained in the pharmaceutical composition of the present invention can carry the nucleic acid contained in the pharmaceutical composition of the present invention and can deliver the nucleic acid into cells, there are no particular limitations on its structure, constituent molecules, or the form in which the nucleic acid is carried. Representative nucleic acid drug delivery systems (DDS) include complexes formed based on electrostatic interactions between positively charged cationic liposomes, cationic polymers, etc., and nucleic acids, which are used as carriers. After binding to the negatively charged cell membrane, the complexes are taken up into cells by adsorptive endocytosis.

More specifically, examples of nucleic acid delivery carriers used in the present invention include, but are not limited to, liposomes (e.g., cationic liposomes, PEG-modified liposomes, etc.), lipid nanoparticles (LNPs), cationic polymers (e.g., polyethyleneimine, polylysine, polyornithine, chitosan, atelocollagen, protamine, etc.), and liposomes containing cationic polymers. Alternatively, exosomes, which are components derived from living organisms, can be used. Preferably, it is a liposome or lipid nanoparticle, more preferably, it is a liposome.

As used herein, a "liposome" refers to a micro closed vesicle having an internal phase surrounded by one or more lipid bilayers, and can usually hold a water-soluble substance in the internal phase and a fat-soluble substance in the lipid bilayer. When the term "encapsulation" is used herein, the nucleic acid of the present invention may be held in the liposomal internal phase or in the lipid bilayer. The liposome used in the present invention may be a single-layer membrane or a multi-layer membrane, and the particle size can be appropriately selected, for example, within the range of 10 to 1000 nm, preferably 50 to 300 nm. In consideration of the delivery to corneal tissue, the particle size can be, for example, 200 nm or less, preferably 100 nm or less.

Preferred components of the liposome used in the present invention include, for example, natural phospholipids such as lecithin, semi-synthetic phospholipids such as dimyristoyl phosphatidylcholine (DMPC), dipalmitoyl phosphatidylcholine (DPPC), and distearoyl phosphatidylcholine (DSPC), dioleyl phosphatidylethanolamine (DOPE), dilauroyl phosphatidylcholine (DLPC), and cholesterol. Other preferred examples include cationic lipids such as N-(α-trimethylammonioacetyl)-didodecyl-D-glutamate chloride (TMAG), N,N',NN",NN‴-tetramethyl-N,N',N",NN"'-tetrapalmitylspermine (TMTPS), 2,3-dioleyloxy-N-[2(sperminecarboxamido)ethyl]-N,N-dimethyl-1-propanaminium trifluoroacetate (DOSPA), N-[1-(2,3-dioleyloxy)propyl]-N,N,N-trimethylammonium chloride (DOTMA), dioctadecyldimethylammonium chloride (DOTMA), dioctadecyldimethylammonium chloride (DODAC), didodecyl ammonium bromide (DDAB), 1,2-dioleyloxy-3-trimethylammoniopropane (DOTAP), 3β-[N-(N',N'-dimethylaminoethane)carbamoyl]cholesterol (DC-Chol), 1,2-dimyristoyloxypropyl-3-dimethylhydroxyethylammonium (DMRIE), and O,O'-ditetradecanoyl-N-(α-trimethylammonioacetyl)diethanolamine chloride (DC-6-14).

Since the nucleic acid contained in the pharmaceutical composition of the present invention exerts its effect by being delivered to myofibroblasts, it is desirable that the nucleic acid delivery carrier of the present invention contains a compound that specifically binds to myofibroblasts in order to more efficiently deliver the nucleic acid of the present invention to myofibroblasts. For example, it is known that myofibroblasts are easy to take up vitamin A. Therefore, it is preferable that the membrane surface of the nucleic acid delivery carrier of the present invention, for example, liposome, is modified with a retinoid derivative that has high binding affinity with vitamin A binding protein and/or vitamin A analogue. The binding or inclusion of the retinoid derivative and/or vitamin A analogue in the nucleic acid delivery carrier of the present invention can also be achieved by binding or inclusion of the retinoid derivative and/or vitamin A analogue in other components of the nucleic acid delivery carrier by chemical and/or physical methods. Alternatively, the binding or inclusion of the retinoid derivative and/or vitamin A analogue in the nucleic acid delivery carrier of the present invention can also be achieved by mixing a retinoid derivative and/or vitamin A analogue that has affinity for formation with the basic nucleic acid delivery carrier components into the components of the nucleic acid delivery carrier when the nucleic acid delivery carrier is produced. The amount of retinoid derivative and/or vitamin A analogue bound to or included in the nucleic acid delivery carrier of the present invention can be 0.01% to 100%, preferably 0.2% to 20%, more preferably 1 to 5% by weight of the nucleic acid delivery carrier components. Nucleic acid delivery carriers containing retinoid derivatives and/or vitamin A analogues and lipids as components are well known to those skilled in the art, for example, the composition disclosed in Japanese Patent No. 6590888.

Methods for encapsulating water-soluble compounds such as nucleic acids into liposomes include, but are not limited to, the lipid film method (vortex method), reverse phase evaporation method, surfactant removal method, freeze-thaw method, remote loading method, etc., and any known method can be appropriately selected.

As used herein, the term "lipid nanoparticle (LNP)" refers to a particle having an average diameter of less than 1 um that does not have a large pore structure (e.g., liposome) or a small pore structure (e.g., mesoporous material) inside the outer shell of a lipid assembly containing cationic lipids and non-cationic lipids.

In one embodiment of the pharmaceutical composition of the present invention, the pharmaceutical composition of the present invention may further contain a drug P other than a nucleic acid that inhibits the expression of mRNA encoding P4HA3. Here, the drug P may be any drug, provided that it does not substantially affect the efficacy of the nucleic acid that inhibits the expression of mRNA encoding P4HA3. For example, the drug P may be an active ingredient of any existing drug for fibrotic diseases. The existing drug for any of the fibrotic diseases may be, for example, a nucleic acid drug such as a nucleic acid that inhibits the expression of mRNA encoding Hsp47, or a non-nucleic acid drug such as pirfenidone. Furthermore, it may be a drug for reducing the side effects of a nucleic acid that inhibits the expression of mRNA encoding P4HA3. As shown in the following examples, the pharmaceutical composition of the present invention exerted a synergistic effect when used in combination with a nucleic acid that inhibits the expression of mRNA encoding Hsp47. Hsp47, like P4HA3, has in common the point of suppressing the expression of mRNA of fibrosis-related factors, but it is thought that the synergistic effect is exerted because the mechanism of action by which the inhibition of mRNA expression of Hsp47 suppresses the expression of mRNA of fibrosis-related factors is different from the mechanism of action by which the inhibition of mRNA expression of P4HA3 suppresses the expression of mRNA of fibrosis-related factors. Other existing drugs for fibrosis diseases, such as pirfenidone, do not suppress the expression of mRNA of fibrosis-related factors, so their mechanism of action is different. Therefore, it is easily expected that the pharmaceutical composition of the present invention will exert a synergistic effect when used in combination with a non-nucleic acid drug such as pirfenidone.

In one embodiment of the pharmaceutical composition of the present invention, the pharmaceutical composition of the present invention can be delivered by at least one parenteral administration selected from the group consisting of subcutaneous administration, intravenous administration, intraarterial administration, intramuscular administration, intraperitoneal administration, intracranial administration, and intrathecal administration. The administration may be continuous or long-term, or short-term or intermittent. The therapeutic agent of the present invention downregulates mRNA encoding P4HA3 and/or reduces intracellular levels of fibrosis-related factors in affected tissues by 50%, 55%, 60%, 65%, 70%, 75%, 80%, 85%, 90%, 95%, or 100% for at least 30 days, at least 35 days, at least 40 days, at least 45 days, at least 50 days, at least 55 days, at least 60 days, at least 65 days, at least 70 days, at least 75 days, at least 80 days, at least 85 days, at least 90 days, at least 95 days, at least 100 days, at least 105 days, at least 110 days, at least 115 days, at least 120 days, or at least 1 year after administration.

The pharmaceutical composition of the present invention may be administered orally, for example, in the form of tablets, capsules, granules, powders, or syrups. These preparations may contain excipients (e.g., organic excipients such as sugar derivatives such as lactose, sucrose, glucose, mannitol, and sorbitol; starch derivatives such as corn starch, potato starch, alpha starch, and dextrin; cellulose derivatives such as crystalline cellulose; gum arabic; dextran; and pullulan: and inorganic excipients such as silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), lubricants (for example, stearic acid metal salts such as stearic acid, calcium stearate, and magnesium stearate; talc; colloidal silica; waxes such as Veegum and Gay wax; boric acid; adipic acid; sulfates such as sodium sulfate; glycol; fumaric acid; sodium benzoate; DL-leucine; fatty acid sodium salts; lauryl sulfates such as sodium lauryl sulfate and magnesium lauryl sulfate; silicic acids such as silicic anhydride and silicic acid hydrate; and the above-mentioned starch derivatives), binders (e.g., hydroxypropylcellulose, hydroxypropylmethylcellulose, polyvinylpyrrolidone, macrogol, and compounds similar to the excipients described above), disintegrants (e.g., cellulose derivatives such as low-substituted hydroxypropylcellulose, carboxymethylcellulose, carboxymethylcellulose calcium, and internally crosslinked sodium carboxymethylcellulose; chemically modified starches and celluloses such as carboxymethylstarch, carboxymethylstarch sodium, and crosslinked polyvinylpyrrolidone), stabilizers (e.g., paraoxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid), and flavoring agents (e.g., commonly used sweeteners, acidulants, and flavors, etc.) are used to produce the formulations by known methods.

The active ingredient contained in the pharmaceutical composition of the present invention is a nucleic acid that inhibits the expression of the mRNA encoding P4HA3 (when the nucleic acid is bound or associated with a nucleic acid delivery carrier, the nucleic acid bound or associated with the nucleic acid delivery carrier). The proportion of the active ingredient contained in the pharmaceutical composition of the present invention can be appropriately set within a range that can produce the desired effect, but is usually 0.01 to 100% by weight, preferably 0.1 to 99.9% by weight, and more preferably 0.5 to 99.5% by weight.

The dosage of the pharmaceutical composition of the present invention must be carefully adjusted taking into account the age, weight, and condition of the individual to be treated, as well as the route, form, and method of administration, and the exact dosage must be determined by a physician. The actual dosage is within the physician's discretion and may vary by setting the dosage for the specific situation of the present invention to obtain the desired therapeutic effect. However, the dosage of the pharmaceutical composition of the present invention is not necessarily determined by the type of the active ingredient, the weight, age, symptoms, etc. of the subject, but for example, in the case of parenteral administration, the lower limit is 0.001 mg/kg body weight (preferably 0.01 mg/kg body weight) and the upper limit is 100 mg/kg body weight (preferably 10 mg/kg body weight), and in the case of oral administration, the lower limit is 0.01 mg/kg body weight (preferably 0.1 mg/kg body weight) and the upper limit is 1000 mg/kg body weight (preferably 100 mg/kg body weight), and it is desirable to administer once or several times a day depending on the symptoms.

As the desirable therapeutic effect to be used in determining the selection of the active ingredient, carrier, dosage, and method of administration of the pharmaceutical composition of the present invention, the inhibition and/or alleviation of the progression of clinical symptoms of fibrotic diseases (physical hardening of the affected tissues). However, as a surrogate endpoint preceding clinical symptoms, the reduction and/or disappearance of fibrosis-related factors accumulated in diseased tissues can also be used to determine the selection of the active ingredient, carrier, administration dose and method of administration of the pharmaceutical composition of the present invention.

One embodiment of the present invention is a method for preventing or treating fibrotic diseases, comprising administering an effective amount of the pharmaceutical composition of the present invention to a subject suffering from or at risk of suffering from a fibrotic disease. Here, administering to a subject at risk of suffering from a fibrotic disease means administering the pharmaceutical composition of the present invention to a subject suffering from myocardial infarction and inflammatory diseases of the liver, lungs, kidneys and the like, which are causes of fibrotic diseases, and who has not yet reached a stage of developing fibrotic diseases. As explained in the examples of the present specification, the pharmaceutical composition of the present invention suppresses the mRNA expression of fibrosis-related factors by suppressing the mRNA expression of P4HA3 produced by myofibroblasts, and therefore fibrotic diseases can be prevented by administering the pharmaceutical composition of the present invention to a subject at a stage of not yet developing fibrotic diseases.

In the present specification, the conjunction "about" used to modify a numerical value means a numerical range of 90% or more and 110% or less of the numerical value. For example, "about 40%" refers to a percentage of a numerical range of 36% or more and 44% or less.

All documents referred to in the present specification are incorporated herein by reference in their entirety.

The examples of the present invention described below are for illustrative purposes only and do not limit the technical scope of the present invention. The technical scope of the present invention is limited only by the claims. Modifications of the present invention, such as additions, deletions, and substitutions of constituent elements of the present invention, may be made without departing from the spirit of the present invention.

### [Example]

### Example 1: Search for genes specifically expressed in myofibroblasts

First, the inventors of the present invention used an animal model of experimental myocardial infarction to search for proteins whose expression levels were significantly increased in the hearts of the myocardial infarction-treated group compared to the sham-treated group, and further narrowed down the molecules whose expression levels in myofibroblasts varied depending on the surrounding stiffness (mechanical stimulation).

The mice used in the examples of the present specification were pure C57BL/6J mice purchased from Japan SLC Co., Ltd., and male mice were 8 to 10 weeks old and female mice were 6 to 8 weeks old. NASH model mice fed an ultra-high fat, choline-deficient, methionine-reduced diet and mice fed a normal diet were created from wild type mice. RNA was then collected from the livers of each mouse, RNA-seq was performed, and the mRNA expression levels of the genes were compared. The present inventors aimed to identify genes that are not expressed at all in normal livers and whose expression levels are increased in NASH livers. First, 1501 kinds of genes were selected as genes that met the criteria that the TPM (transcripts per million) value in mice fed normal chow was 0 and the TPM value in the liver of the NASH model mouse group was greater than 0.1. Next, the inventors aimed to identify genes that are highly expressed in myofibroblasts in the liver. Therefore, myofibroblasts were isolated from the liver of fibrotic mice, RNA-seq was performed, and genes with a TPM value of greater than 50 were selected. As a result, 19 kinds of genes were selected. Of these, genes that were likely to be specifically expressed in myofibroblasts were selected. As a result of a literature search, etc., it was found that P4HA3 is specifically expressed in myofibroblasts.

### Example 2: Specific expression of P4HA3 mRNA in a disease model mouse of myocardial infarction

A disease model mouse of myocardial infarction was prepared as described in Nakaya, M. et al. (J Clin Invest. 2017; 127(1):383-401.). Briefly, to induce acute myocardial infarction in mice, the mice were anesthetized with intraperitoneal injection of Somnopentyl injection (50 mg/kg sodium pentobarbital) and a cannula was inserted into the trachea. The left intercostal space was incised to open the chest, the heart was aspirated and removed from the thoracic cavity, and the left anterior descending coronary artery was ligated with 6 mm silk braided suture. In the sham treatment group, the chest was opened and the heart was removed from the thoracic cavity but was not ligated. In both the treatment and sham treatment groups, the heart was returned to the thoracic cavity and the chest was closed.

The mRNA expression levels of P4HA3 etc. in the examples of the present specification were quantified as follows. The concentration of purified RNA was determined using a microspectrophotometer DS-11 (DeNovix), and reverse transcription to cDNA was performed using a High Capacity cDNA Reverse Transcription Kit (Applied Biosystems). Quantification of mRNA was performed according to the protocol of Luna Universal qPCR Master Mix (New England BioLabs), with DNA polymerase activation at 95°C for 60 seconds, followed by 45 cycles of cDNA denaturation at 95°C for 15 seconds and extension at 60°C for 30 seconds. Analysis was performed by the ΔΔCt method using GAPDH as an internal standard. Mouse Cyp7a1 probe was purchased from Applied Biosystems. Primers and probes for mouse α-smooth muscle actin (α-SMA), CD68, 1α1 collagen (Col1α1), 1α2 collagen (Col1α2), 1α3 collagen (Col1α3), 6α1 collagen (Col6α1), 8α1 collagen (Co81α1), GAPDH (glyceraldehyde-3-phosphate dehydrogenase), heat shock protein 47 (Hsp47), prolyl 4-hydroxylase alpha1 subunit (P4HA1), prolyl 4-hydroxylase alpha2 subunit (P4HA2), and prolyl 4-hydroxylase alpha3 subunit (P4HA3) were purchased from Sigma-Aldrich.

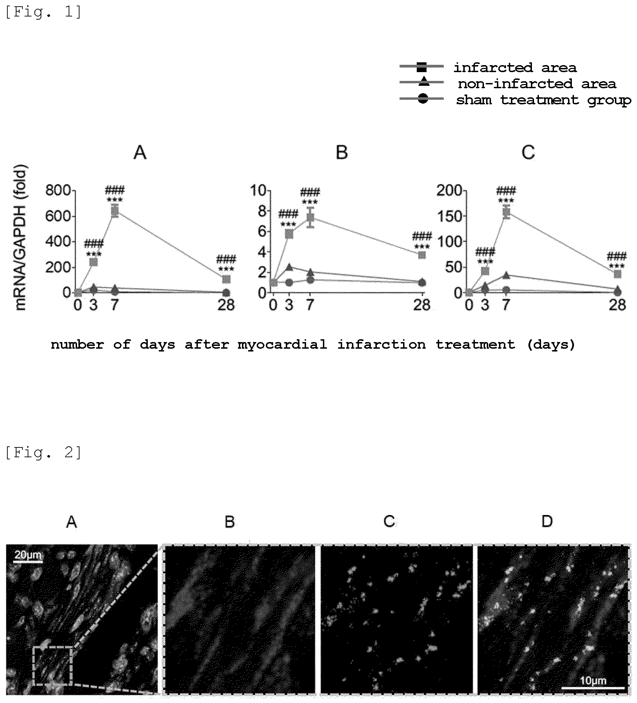

Fig. 1 shows the time-dependent change of the relative mRNA expression levels of P4HA3 (A), α-SMA (B), and periostin (C) in the heart after myocardial infarction. α-SMA is a specific marker for myofibroblasts, and periostin is also an extracellular matrix protein known to be specifically expressed in myofibroblasts. ■ indicates the mRNA expression level of each gene measured by real-time RT-PCR in the infarcted region, ▲ indicates the mRNA expression level of each gene measured by real-time RT-PCR in the non-infarcted region, and ● indicates the mRNA expression level of each gene measured by real-time RT-PCR in the sham treatment group (sham). The results are from 3 to 6 experiments performed under the same conditions. The error bars for the mRNA expression level of each gene in the infarcted are 7 days after treatment represent the standard deviation. In the graph in Fig. 1, the vertical axis indicates the relative value of the mRNA expression level of each gene in the heart sample on day 0 of the sham treatment group, where the value obtained by correcting the mRNA expression level of each gene by the GAPDH gene mRNA expression level is taken as 1, and the horizontal axis indicates the number of days after the myocardial infarction treatment (unit: days). The number of samples in the infarcted region, non-infarcted region, and sham treatment group were 5-6, 3, and 3-4, respectively. P values were calculated by unpaired two-tailed Student's t-test. ### on the graph indicates that the significance of the infarcted area vs. sham treatment group (inf vs. sham) at the same number of days after infarction treatment is p<0.001, and *** on the graph indicates that the significance of the infarcted area vs. non-infarcted area (inf vs. rem) on the same number of days after infarction treatment is p<0.001. Compared with αSMA and periostin, the expression level of P4HA3 mRNA was significantly higher in the infarcted area of the treatment group, and significantly lower in the non-infarcted area of the treatment group and in the sham treatment group. Thus, the results showed that P4HA3 mRNA has higher expression specificity at the site of myocardial infarction, compared with αSMA and periostin.

To identify cells expressing P4HA3 during myocardial infarction, in situ hybridization was performed on heart sections 7 days after myocardial infarction using an RNAscope^{™} reagent kit (Advanced Cell Diagnostics, Cosmo Bio Co., Ltd.). In situ hybridization was performed using an RNAscope from Advanced Cell Diagnostics, following the protocol. The prepared frozen tissue sections were first washed with PBS, after which a protease solution was dripped onto them and reacted at 40°C for 30 min in a HybEZ^{™} oven (Advanced Cell Diagnostics). A probe for P4HA3 was then dripped onto the tissue sections and reacted in a HybEZ^{™} oven at 40°C for 2 hr, after which a signal amplification reaction was performed using Amp solution. The sections were then reacted with fluorescently labeled Tyramide to detect the P4HA3 mRNA signal. After the signal detection reaction, co-staining with antibodies was performed. 5% BSA/PBS was dropped and reacted (blocked) at room temperature for 1 hr, and Anit-Desmin antibody (Abcam, #ab15200) was reacted at 4°C overnight. The next day, fluorescently labeled Alexa Fluor 488(R)-conjugated goat anti-rabbit IgG (Invitrogen, #A-11008) was reacted at room temperature for 1 hr, and the nuclei were stained with DAPI, and the sections were mounted using FluorSaveTM Reagent. Images were taken using a confocal microscope (LSM700, Zeiss).

Fig. 2 is a panel of fluorescent microscopy images showing the results of in situ hybridization of heart sections on 7 days after myocardial infarction. A is a fluorescence microscopy image obtained by merging a fluorescence microscopy image showing the results of in situ hybridization using periostin and P4HA3 probes with a DAPI-stained fluorescence microscopy image. B, C, and D are all enlarged views of the area surrounded by the dashed line in A. B is a fluorescence microscopy image showing the results of in situ hybridization using a periostin probe, C is a fluorescence microscopy image showing the results of in situ hybridization using a P4HA3 probe, and D is a fluorescence microscopy image obtained by merging the fluorescence microscopy images of B and C. As shown in Fig. 2, P4HA3 mRNA is specifically expressed in myofibroblasts, like periostin mRNA, but whereas periostin mRNA is distributed almost uniformly in the cytoplasm of myofibroblasts, P4HA3 mRNA is scattered in clumps in part of the cytoplasm of myofibroblasts.

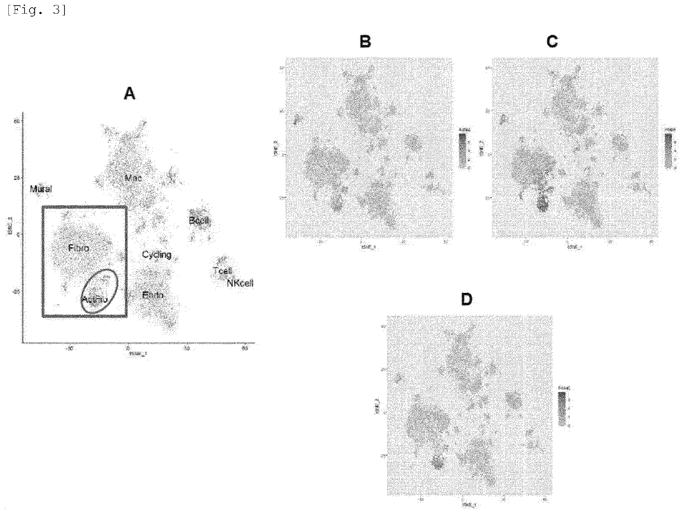

The fact that P4HA3 mRNA is specifically expressed in myofibroblasts was also confirmed by single-cell analysis of each cell type in mouse hearts before and after treatment in a known myocardial infarction model. Fig. 3 is a t-SNE plot created using the data from Farbehi N. et al. (2019) above, showing the results of single-cell analysis of each cell type in mouse hearts before and after treatment in a myocardial infarction model. A is a dimensionality reduction plot of cell populations expressing each cell type-specific mRNA, B is a dimensionality reduction plot visualizing cell populations expressing the αSMA (Acta2) gene, C is a dimensionality reduction plot visualizing cell populations expressing the periostin (Postn) gene, and D is a dimensionality reduction plot visualizing cell populations expressing the P4HA3 gene. Mac indicates macrophages, End indicates endothelial cells, Cycling indicates proliferating endothelial cells, Mural indicates smooth muscle cells and pericytes, Fibre indicates fibroblasts, and ActFibro indicates activated fibroblasts.

### Example 3: Effect of P4HA3 knockdown on the expression of each gene in myofibroblasts derived from myocardial infarction sites

Next, the inventors investigated the effect of suppressing P4HA3 mRNA expression on the mRNA expression of other fibrosis-related factors. Myofibroblasts from the infarcted area of myocardial infarction disease model mice were cultured as described above in Nakaya, M. et al. (2017). Briefly, 3 days after coronary artery ligation, the ventricles of treated mice were digested with PBS containing 0.1% collagenase A (Roche Diagnostics) and 0.1% trypsin (Sigma-Aldrich) at 37°C with agitation. The cells were placed on plates containing 10% FBS/DMEM overnight, and then the medium was replaced to remove non-adherent cells. The attached cells were cultured for more than 6 days and used as myofibroblasts in the following experiments. The myofibroblasts used were from passage 1 (P1). The myofibroblasts were transfected with siRNA using Lipofectamine 2000 (Invitrogen). The si P4HA3 used was ThermoFisher Scientific catalog number 4390771 (Assay ID: s115723) and was added to the myofibroblast culture at a final concentration of 3.125 nM. The si Ctrl used as a control siRNA was ThermoFisher Scientific catalog number 4390843 and was added to the myofibroblast culture at a final concentration of 3.125 nM. The quantification of mRNA expression levels of P4HA3, etc. in myofibroblasts after siRNA treatment was performed in the same manner as in Example 2.

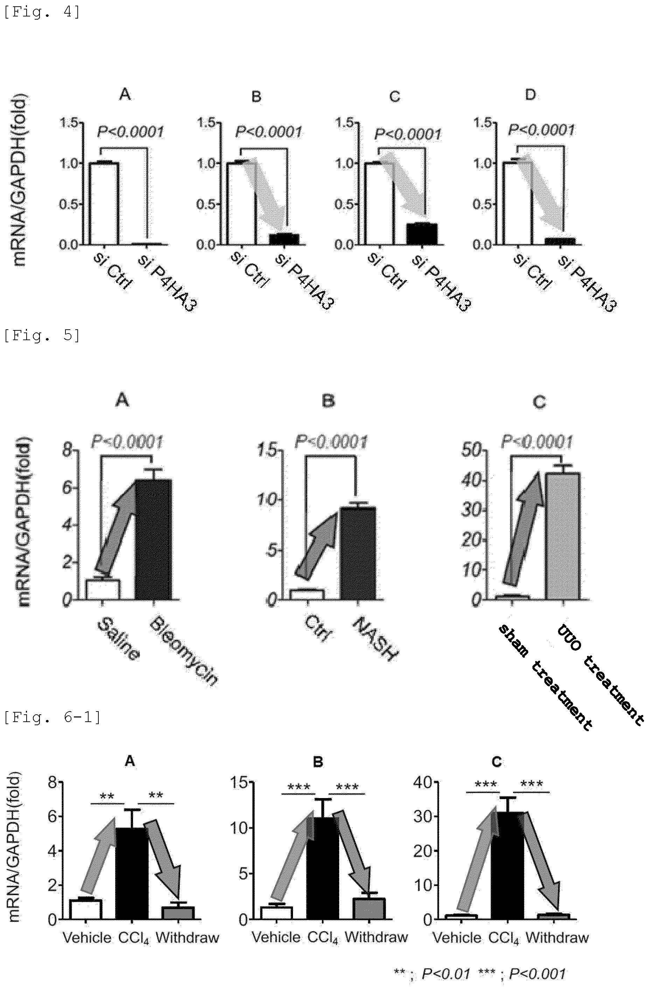

Fig. 4 is a bar graph showing the effect of siRNA against the P4HA3 gene on the expression of the P4HA3 (A), periostin (B), 1α1 collagen (C), and 3α1 collagen (D) genes in myofibroblasts isolated from the infarcted region, si P4HA3 and si Ctrl represent the relative values of the mRNA expression levels of each gene measured by real-time RT-PCR in myofibroblasts administered with siRNA against the P4HA3 gene and siRNA against a control gene, respectively. The vertical axis represents the relative value of the mRNA expression level of each gene in the si Ctrl group, where the value obtained by correcting the mRNA expression level of each gene by the GAPDH gene mRNA expression level is taken as 1. The number of samples is 5. P values were calculated by unpaired two-tailed t-test. Error bars indicate standard deviation.

As shown in Fig. 4, myofibroblasts treated with si P4HA3 (the same siRNA as si P4HA3#1 in Example 7) strongly inhibited not only the expression of P4HA3 mRNA, but also the expression of periostin, 1α1 collagen, and 3α1 collagen mRNA. The above P4HA3 knockdown experiment suggested that P4HA3 functions as an essential regulator of the transcription of periostin, 1α1 collagen, and 3α1 collagen in myofibroblasts differentiated after myocardial infarction.

### Example 4: Involvement of P4HA3 in fibrosis in other organs

To confirm that the involvement of P4HA3 in fibrosis is not limited to myocardial infarction, the following experiments were performed using disease model mice with pulmonary fibrosis, fatty liver, and chronic renal failure. The amount of mRNA expression of P4HA3, etc. was quantified in the same manner as in Example 2.

A mouse model of pulmonary fibrosis was created by anesthetizing 6-8 week old female mice with isoflurane inhalation and administering saline containing bleomycin intrabronchially. 1 mg/kg was administered for samples from which lung tissue mRNA was extracted, and 1.5 mg/kg was administered for other tissue section preparations, myofibroblast isolation, and viability measurements. Physiological saline alone was injected into the sham treatment group. Lung RNA was collected 14 days after administration of bleomycin or saline alone.

A mouse model of liver fibrosis was created by administering carbon tetrachloride or feeding an ultra-high fat, choline-deficient, methionine-reduced diet. When carbon tetrachloride (0.4 mL per kg body weight) was diluted 5-fold with corn oil and administered intraperitoneally twice a week for 4 weeks, signs of liver fibrosis were observed. However, when carbon tetrachloride administration was discontinued and the mice were kept for another 4 weeks, signs of liver fibrosis disappeared. In mice with NASH induced by carbon tetrachloride, liver RNA was collected after 4 weeks of carbon tetrachloride administration (CCl₄ group), 4 weeks of corn oil administration (Vehicle group) as a control, or 4 weeks after the withdrawal of carbon tetrachloride administration (Withdrawal group). The Vehicle and CCL4 groups were anesthetized with Somnopentyl 24 hr after the final administration, and Withdrawal group 4 weeks after the final administration, and the abdomen and chest were opened, and the liver was perfused with PBS by cardiac puncture, and after confirming that blood had been removed from the liver, the liver was excised. Total RNA was then collected according to the protocols of Isogen (Nippon Gene) and RNeasyPlus Mini Kit (Qiagen). Diet-induced NASH model mice were fed an ultra-high fat, choline-deficient, methionine-reduced diet (60 kcal% fat content, lard used, choline-deficient, methionine-reduced (0.1%), CDAHFD (ResearchDiets)) for 5 weeks, then switched back to normal diet (SD (Research Diets)) for another 5 weeks, after which liver RNA was collected.

Chronic renal failure model mice were created by unilateral ureteral obstruction (UUO) according to Chevalier, R.L. et al. (Kidney International (2009) 75, 1145-1152). 8-10 week old female mice were anesthetized by inhalation of 1.5% isoflurane and fixed on the operating table in a supine position while maintaining inhalation anesthesia. Under a surgical microscope, the abdomen was incised in the midline, the right ureter was exposed, and the ureter was ligated with 6-0 silk braided suture. Then, 30 µL of procaine penicillin G solution was dripped into the abdominal cavity, and the incision was sutured with suture. Ten days after UUO, the animals were anesthetized with Somnopentyl, the abdomen was opened, the abdominal aorta was cut, and blood was removed. The kidney was then removed and total RNA was collected. The kidney on the opposite side, which had not been subjected to UUO, was used as a sham treatment group.

Fig. 5 is a bar graph showing the mRNA expression level of the P4HA3 gene measured by real-time RT-PCR in an experimental fibrosis model of the lung (A), liver (B), and kidney (C). Graph A shows the relative expression level of P4HA3 mRNA in lung tissues of the bleomycin-treated group (Bleomycin) or sham-treated group (Saline), graph B shows the relative expression level of P4HA3 mRNA in liver tissues of the ultra-high fat, choline-deficient, methionine-reduced diet group (NASH) or normal diet-fed group (Ctrl), and bar graph C shows the relative expression level of P4HA3 mRNA in kidney tissues of the UUO-treated group (UUO) or sham-treated group. The vertical axis of each bar graph shows the relative expression level of P4HA3 mRNA, where the value corrected by the expression level of GAPDH gene mRNA is taken as 1. P values were calculated by unpaired two-tailed t-test.

As shown in Fig. 5, in all organs of fibrosis model mice, the expression of P4HA3 mRNA in the treatment group was several to several tens of times higher than that in the sham-treated group. Thus, it was revealed that expression of P4HA3 increases not only in the heart but also in the lungs, liver, and kidneys during fibrotic pathology.

### Example 5: Time course of mRNA expression of P4HA3 and fibrosis-related factors in livers with transient fibrosis

Next, the mRNA expression of P4HA3 and fibrosis-related factors was examined using carbon tetrachloride-administered transient liver fibrosis model mice and diet-administered NASH model mice.

Fig. 6-1 is a bar graph showing time course of relative mRNA expression levels of P4HA3 (A), periostin (B), and 1α1 collagen (C) measured by real-time RT-PCR in liver tissues collected from carbon tetrachloride-administered transient liver fibrosis mice model at 4 weeks after the start of vehicle administration (Vehicle), at 4 weeks after the start of carbon tetrachloride administration (CCl₄), and at 4 weeks after the cessation of carbon tetrachloride administration (Withdraw). The vertical axis of each bar graph shows the relative value of the mRNA expression level of each gene, where the value of the mRNA expression level of each gene in the group 4 weeks after the start of solvent administration (Vehicle) corrected by the mRNA expression level of GAPDH is set to 1. The results are from five experiments performed under the same conditions.

As shown in Fig. 6-1, in livers where transient liver fibrosis had occurred due to carbon tetrachloride administration, the expression of P4HA3, α-SMA, and 1α1 collagen mRNAs was increased compared to the Vehicle group, but when carbon tetrachloride administration was discontinued, the expression decreased to almost the same level as the Vehicle group.

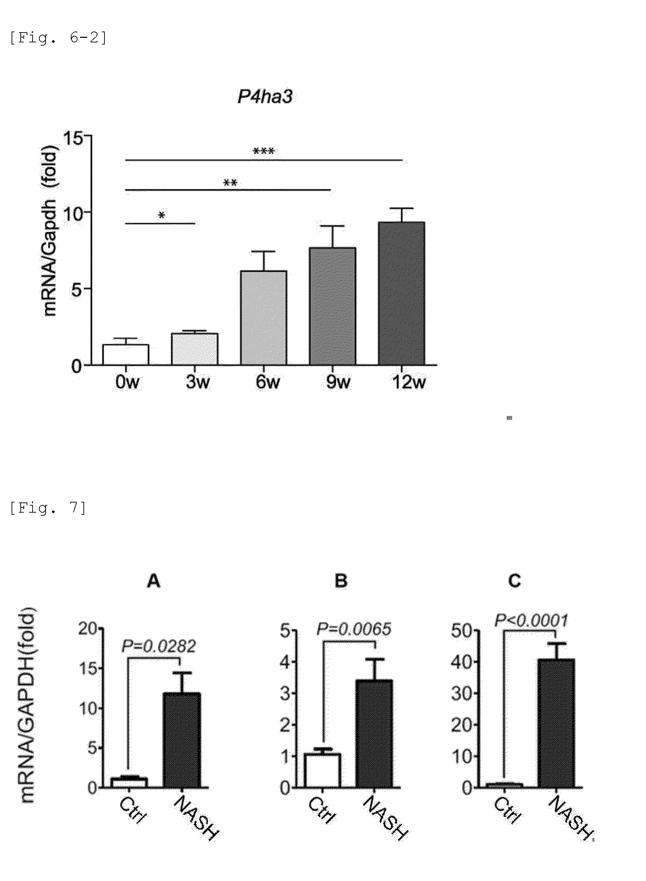

Fig. 6-2 is a bar graph showing the time course of the expression level of P4HA3 mRNA in the liver of diet-induced NASH model mice. Each bar graph (0W, 3W, 6W, 9W, and 12W) shows the time course of the relative mRNA expression level of P4HA3 measured by real-time RT-PCR in liver tissue collected from mice before, 3 weeks after, 6 weeks after, 9 weeks after, and 12 weeks after feeding an ultra-high fat, choline-deficient, methionine-reduced diet. The vertical axis of each bar graph shows the relative value of the P4HA3 mRNA expression level at each time point, where the value obtained by correcting the P4HA3 mRNA expression level before feeding with the GAPDH mRNA expression level is set to 1. The results are from six experiments conducted under the same conditions (five times only after 3 weeks of feeding).

As shown in Fig. 6-2, in livers with fibrosis caused by an ultra-high fat, choline-deficient, methionine-reduced diet, P4HA3 mRNA expression tends to increase with the length of feeding period, with a particularly large difference between 3-week and 6-week feeding periods.

Fig. 7 is a bar graph showing the mRNA expression levels of each gene measured by real-time RT-PCR in diet-induced NASH model mice. Each bar graph shows the mRNA expression levels of P4HA3 (A), α-smooth muscle actin (α-SMA) (B), and 1α 1 collagen (C) in liver tissues from mice with NASH fed an ultra-high fat, choline-deficient, methionine-reduced diet for 5 weeks and collected 5 weeks after the diet was stopped (NASH), and in liver tissues from control mice fed a normal diet (Ctrl). The vertical axis shows the relative value of the mRNA expression level of each gene in the Ctrl group, where the value obtained by correcting the mRNA expression level of each gene by the GAPDH gene mRNA expression level is set to 1. The P value was calculated by unpaired two-tailed t-test.

As shown in Fig. 7, the expression of P4HA3, α-SMA, and 1α1 collagen mRNA was increased compared to the Ctrl group in the diet-fed NASH model mice.

Here, α-SMA is a marker for myofibroblasts, and 1α1 collagen is one of the fibrosis-related factors produced by myofibroblasts. Figs. 6-1, 6-2, and 7 suggest that in liver fibrosis, as in myocardial infarction, myofibroblasts appear and produce fibrosis-related factors, and P4HA3 may function as an essential regulator for the transcription of the fibrosis-related factors.

### Example 6: Gene expression in each fraction of liver with transient fibrosis

Using a mouse model of transient liver fibrosis caused by carbon tetrachloride administration, the inventors investigated whether P4HA3 is expressed only in myofibroblasts.

From a mouse model of transient liver fibrosis caused by carbon tetrachloride administration, the liver was removed 4 weeks after the start of carbon tetrachloride administration. The liver was treated with collagenase A at 37°C for 30 min, and the cell suspension was centrifuged at 50G for 1 min to isolate the precipitated cells as a hepatocyte fraction. Meanwhile, the supernatant was seeded on a plastic plate and cultured overnight in DMEM supplemented with 10% FBS, after which the cells attached to the plate were collected using Accutase, and the CD45-positive fraction was separated as a blood cell fraction using CD45 antibody-added magnetic beads. At the same time, the CD45-negative fraction was identified as a myofibroblast fraction. The mRNA expression levels of Cyp7a1, a marker for hepatocytes, Cd68, a marker for blood cells, α-SMA, a marker for myofibroblasts, and P4HA3 were quantified in the same manner as in Example 2.

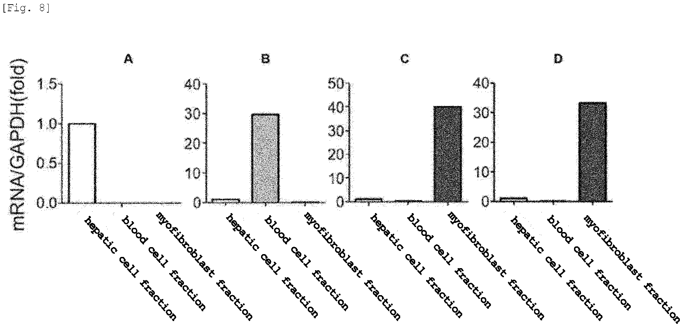

Fig. 8 is a bar graph showing the relative values of mRNA expression levels measured by real-time RT-PCR for the Cyp7a1 (A), Cd68 (B), α-SMA (C), and P4HA3 (D) genes for each cell type fraction of the liver collected 4 weeks after the start of carbon tetrachloride administration. The vertical axis represents the relative value of the mRNA expression level of each gene, where the value obtained by correcting the mRNA expression level of each gene in the hepatocyte fraction by the mRNA expression level of GAPDH is taken as 1.

As shown in Fig. 8, the mRNA expression of Cyp7a1 was detected only in the hepatocyte fraction, the mRNA expression of Cd68 was detected almost exclusively in the blood cell fraction, and the mRNA expression of α-SMA and P4HA3 was detected almost exclusively in the myofibroblast fraction. Therefore, it was shown that P4HA3 is specifically expressed in myofibroblasts even in fibrotic livers.

### Example 7: Effect of P4HA3 knockdown on the expression of fibrosis-related factors in myofibroblasts derived from transiently fibrotic livers

As in Example 3, the effect of suppressing P4HA3 mRNA expression on the mRNA expression of other fibrosis-related factors was examined in cultured myofibroblasts isolated from the liver 4 weeks after the start of carbon tetrachloride administration. The liver was removed from a mouse model of carbon tetrachloride (CCl₄)-induced fibrotic livers and divided into small pieces. After that, the liver pieces were washed with washing buffer [25 mM HEPES/500 nM EDTA/HBSS(-)], and the cell solution containing myofibroblasts was collected by shaking the liver pieces three times for 30 min at 37°C in digestion buffer [0.1% Collagenase A/15 mM HEPES/HBSS(-)]. After that, blood cell components were removed with Red Blood Cell Lysis Buffer, and the cells were suspended in 10% FBS/1% Penicillin-Streptomycin/DMEM, and then seeded on a culture plate. After 6 hr, the medium was replaced to remove non-adherent cells and debris. After that, the cells were cultured overnight in a CO₂ incubator, and non-adherent cells were again removed by washing with PBS, and adherent cells were collected using Accutase. After 20 min of reaction with CD45 antibody labeled with magnetic beads, CD45-positive blood cells were removed by MACS (magnetic cell sorting) using an LS column, and CD45-negative cells were collected as myofibroblasts. After that, the purity of the isolated myofibroblasts was measured using FACS verse, and almost all of the CD45-negative cells after MACS were positive for α-SMA, a myofibroblast marker, and almost no contamination with blood cells was observed. Si P4HA3#1 and si P4HA3#2, ThermoFisher Scientific catalog numbers 4390771 and 4390771 (Assay ID: s115723 and s115721), were added to the myofibroblast culture at a final concentration of 3.125 nM using Lipofectamine RNAiMAX (Invitrogen). In experiments in which si P4HA3#2 was not used, such as in Example 3, si P4HA3#1 was used as "si P4HA3". For siRNA control experiments, Silencer Select Negative Control no. 1 siRNA (ThermoFisher Scientific, catalog number: 4390843) was used as si Ctrl. The nucleotide sequences of the sense and antisense strands of si P4HA3#1 are listed in the sequence listing attached hereto as SEQ ID NOs: 1 and 2, respectively. The nucleotide sequences of the sense and antisense strands of si P4HA3#2 are listed in the sequence listing attached hereto as SEQ ID NOs: 3 and 4, respectively.

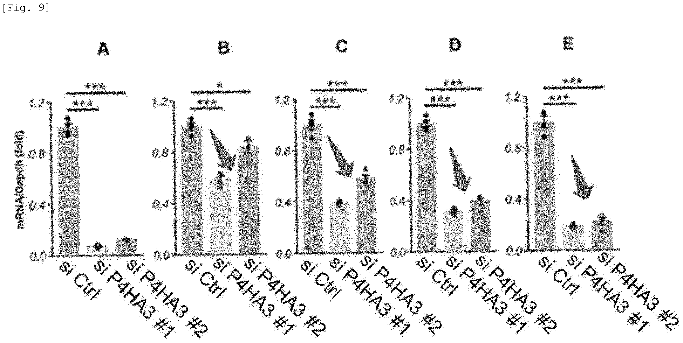

Fig. 9 is a bar graph showing the effect of P4HA3 knockdown on the expression of the P4HA3 gene (A), 1α1 collagen (B), 1α2 collagen (C), 3α1 collagen (D), and elastin (E) genes in myofibroblasts isolated from the liver of a liver fibrosis model mouse. The left bar of each bar represents the relative expression level of each gene in myofibroblasts administered with si Ctrl, the right bar represents si P4HA3#1, and the center bar represents the relative expression level of each gene in myofibroblasts administered with si P4HA3#2. The vertical axis shows the relative value of the mRNA expression level of each gene, where the value obtained by correcting the mRNA expression level of each gene in the si Ctrl group by the GAPDH gene mRNA expression level is taken as 1. The results are from four experiments performed under the same conditions. * indicates P<0.05, *** indicates P<0.001. The P value was calculated by Tukey's test. The error bars indicate the standard deviation.

As shown in Fig. 9, si P4HA3#1 and si P4HA3#2 inhibited the mRNA expression of P4HA3, 1α1 collagen, and other fibrosis-related factors in myofibroblasts derived from transiently fibrotic liver. However, the inhibitory effect was weaker than the inhibition of the mRNA expression of fibrosis-related factors in myofibroblasts derived from myocardial infarction sites shown in Fig. 4 of Example 3. In addition, for all mRNA expression, si P4HA3#1 had a stronger inhibitory activity than si P4HA3#2. The difference in the mRNA expression inhibitory effect of si P4HA3#1 and #2 was greater for 1α1 collagen and 1α2 collagen than for P4HA3. In contrast, there was no significant difference in the mRNA expression inhibitory effect of si P4HA3#1 and si P4HA3#2 for 3α1 collagen and elastin. This suggests that the effect of P4HA3 knockdown on the mRNA expression of other fibrosis-related factors in myofibroblasts is not uniform, and that there are multiple mechanisms by which P4HA3 knockdown inhibits the mRNA expression of fibrosis-related factors, which may differ for each fibrosis-related factor (or its subgroup).

### Example 8: mRNA expression of P4HA3 and fibrosis-related factors in fibrotic lungs

In a bleomycin-administered pulmonary fibrosis model mouse prepared in the same manner as in Example 4, the mRNA expression of P4HA3 and several other fibrosis-related factors was examined. The mRNA expression levels of P4HA3, etc. were quantified in the same manner as in Example 2.

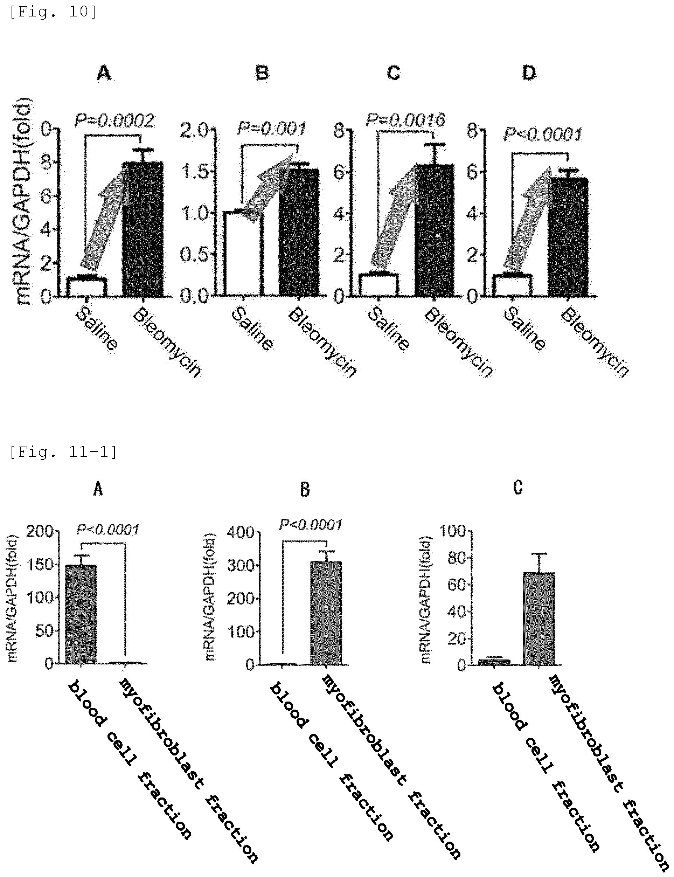

Fig. 10 is a bar graph showing the relative values of mRNA expression levels measured by real-time RT-PCR for the P4HA3 (A), α-SMA (B), 1α1 collagen (C), and 3α1 collagen (D) genes in lung tissue from mice with pulmonary fibrosis, collected 7 days after intrabronchial administration of bleomycin (bleomycin), and in lung tissue from control mice administered physiological saline (saline). The vertical axis of each graph represents the relative value of the mRNA expression level of each gene, where the value obtained by correcting the mRNA expression level of each gene in the saline group by the mRNA expression level of GAPDH is taken as 1.

As shown in Fig. 10, even in the bleomycin-induced pulmonary fibrosis model, the mRNA expression levels of P4HA3, α-SMA, 1α1 collagen, and 3α1 collagen were higher in the treatment group than in the sham treatment group. Compared to Fig. 7, the mRNA expression of P4HA3 and 1α1 collagen was increased in the lungs of the pulmonary fibrosis model mice, similar to that in the liver of the NASH model mice in Fig. 7. However, α-SMA was only slightly increased in the lungs of the pulmonary fibrosis model mice compared to the liver of the NASH model mice. This is because, in the NASH model mice shown in Fig. 7, mRNA expression was examined in livers collected 10 weeks after the start of feeding the ultra-high fat, choline-deficient, methionine-reduced diet, whereas in the bleomycin-administered pulmonary fibrosis model shown in Fig. 10, mRNA expression was examined in lungs collected 7 days after intrabronchial administration of bleomycin. This suggests the possibility that the increase in the amount of mRNA expression of α-SMA, a marker for myofibroblasts, was small because the time from the start of fibrosis induction was short and not many myofibroblasts had differentiated.

### Example 9: Gene expression in each fraction of lungs that had developed fibrosis due to bleomycin administration

Using a bleomycin-administered pulmonary fibrosis model mouse, the inventors investigated whether P4HA3 was expressed only in myofibroblasts.

From the bleomycin-administered pulmonary fibrosis model mouse prepared in the same manner as in Example 4, the lungs were removed 7 days after bleomycin administration. The liver was treated with collagenase A at 37°C for 30 min, and the cell suspension was separated by FACSAria. The resulting CD45-positive fraction was used as the blood cell fraction, and the PDGFRα-positive fraction was used as the myofibroblast fraction. Quantification of the mRNA expression levels of Cd68, a blood cell marker, 1α1 collagen, a fibrosis-related factor, and P4HA3 was performed in the same manner as in Example 2.

Fig. 11-1 is a bar graph showing the relative values of the mRNA expression levels measured by real-time RT-PCR for the genes Cd68 (A), 1α1 collagen (B), and P4HA3 (C) in the blood cell fraction and myofibroblast fraction, respectively, isolated as the CD45-positive fraction and PDGFRα-positive fraction by FACSAria from the lungs of a bleomycin-administered pulmonary fibrosis model mouse. The vertical axis shows the relative value of the mRNA expression level of each gene, where the value obtained by correcting the mRNA expression level of each gene in the hepatic cell fraction by the mRNA expression level of GAPDH is taken as 1.

From Fig. 11-1(A), the blood cell fraction isolated as the CD45-positive fraction expresses Cd68, a marker of blood cell, very strongly, but the myofibroblast fraction isolated as the PDGFRα-positive fraction hardly expresses Cd68. Conversely, the myofibroblast fraction expresses 1α1 collagen, a fibrosis-promoting factor, very strongly, but the blood cell fraction hardly expresses 1α1 collagen. Furthermore, P4HA3 is expressed very strongly in the myofibroblast fraction, but is hardly expressed in the blood cell fraction. These results demonstrate that P4HA3 is expressed in myofibroblasts even in fibrotic lungs.

### Example 10: Effect of P4HA3 knockdown on expression of fibrosis-related factors in myofibroblasts derived from fibrotic lungs

The effect of P4HA3 knockdown on mRNA expression of fibrosis-related factors was examined in the same manner as in Example 3, using myofibroblasts fractionated from bleomycin-administered pulmonary fibrosis model mice.

Fig. 11-2 is a bar graph showing the effect of siRNA administration on the expression of P4HA3 (A), 1α1 collagen (B), 3α1 collagen (C), and 14α1 collagen (D) genes in myofibroblasts isolated from the lungs of bleomycin-administered pulmonary fibrosis model mice and administered si Ctrl or si P4HA3. The left bar of each bar represents the relative value of the mRNA expression level of each gene in myofibroblasts administered si Ctrl and the right bar represents the relative value of the mRNA expression level of each gene in myofibroblasts administered si P4HA3. The vertical axis shows the relative value of the mRNA expression level of each gene, where the value of the mRNA expression level of each gene in the si Ctrl group corrected by the mRNA expression level of GAPDH is taken as 1. The results are from five experiments performed under the same conditions. The error bars show the standard deviation.

As shown in Fig. 11-2, P4HA3 mRNA expression was very strongly inhibited in myofibroblasts administered with si P4HA3. The mRNA expression levels of 1α1 collagen, 3α1 collagen, and 14α1 collagen in myofibroblasts administered with si P4HA3 were reduced to a fraction of their original levels. Compared with Example 3 using myofibroblasts derived from myocardial infarction model mice and Example 7 using myofibroblasts derived from liver fibrosis model mice, in myofibroblasts derived from pulmonary fibrosis model mice, the mRNA expression level of P4HA3 was reduced to less than 5% by siRNA of the P4HA3 gene, but the mRNA expression level of fibrosis-related factors was reduced to 25% to 50%, but the mRNA expression level was not reduced as much as that of P4HA3. This result suggests that the mechanism of action by which inhibition of P4HA3 mRNA expression causes inhibition of fibrosis-related factor mRNA expression may differ depending on which organ the myofibroblasts are derived from.

### Example 11: Expression of P4HA3 and fibrosis-related factors in fibrotic kidneys

The expression of P4HA3 and fibrosis-related factors in mouse kidneys with fibrosis due to unilateral ureteral obstruction (UUO) was examined by in situ hybridization and fluorescent immunohistochemistry. Fig. 12-1 is a schematic coronal section including the long axis of a kidney with fibrosis due to unilateral ureteral obstruction (UUO). Due to physical pressure from ureteral ligation, the parenchyma consisting of the cortex and medulla is thinner than that of the sham kidney. In addition, the expression of α-SMA is strongest in the region adjacent to the ureter in the renal medulla, and collagen expression is weaker than that of the region adjacent to the cortex in the renal cortex. Conversely, the expression of collagen is strongest in the region adjacent to the cortex in the renal cortex, and α-SMA expression is weaker than that of the region adjacent to the ureter in the renal medulla. i to vi indicate the positions of kidney tissue sections.

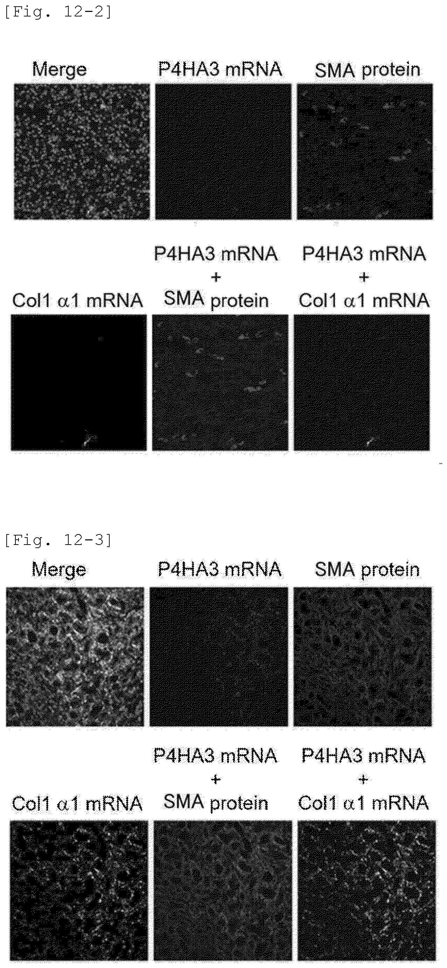

Fig. 12-2 shows fluorescent microscopy images (all at 20x magnification) of a sham-treated kidney tissue section (sham) corresponding to area i in Fig. 12-1, showing the results of in situ hybridization using P4HA3 and 1α1 collagen probes, and the results of fluorescent histochemistry using anti-α-SMA antibody. The top center is a fluorescent microscopy image showing the results of in situ hybridization using the P4HA3 probe, the top right is a fluorescent microscopy image showing the results of fluorescent histochemistry using anti-α-SMA antibody, and the top left is a fluorescent microscopy image merging the fluorescent microscopy images in the top center and top right with a DAPI-stained fluorescent microscopy image. The bottom left is a fluorescent microscopy image showing the results of in situ hybridization using the 1α1 collagen probe, the bottom center is a fluorescent microscopy image merging the fluorescent microscopy images in the top center and top right, and the bottom right is a fluorescent microscopy image merging the fluorescent microscopy images in the top center and bottom left.

Fig. 12-3 shows fluorescent microscopy images of renal tissue sections from the UUO-1 region (see area i in Fig. 12-1) on the side where unilateral ureteral obstruction was performed, showing the results of in situ hybridization using P4HA3 and 1α1 collagen probes, and fluorescent microscopy images showing the results of fluorescent histochemistry using anti-α-SMAantibody (all at 20x magnification). The explanation for each image is the same as for Fig. 12-2. The UUO-1 region is the region with the strongest expression of α-SMA, indicating that fibrosis is highly advanced.

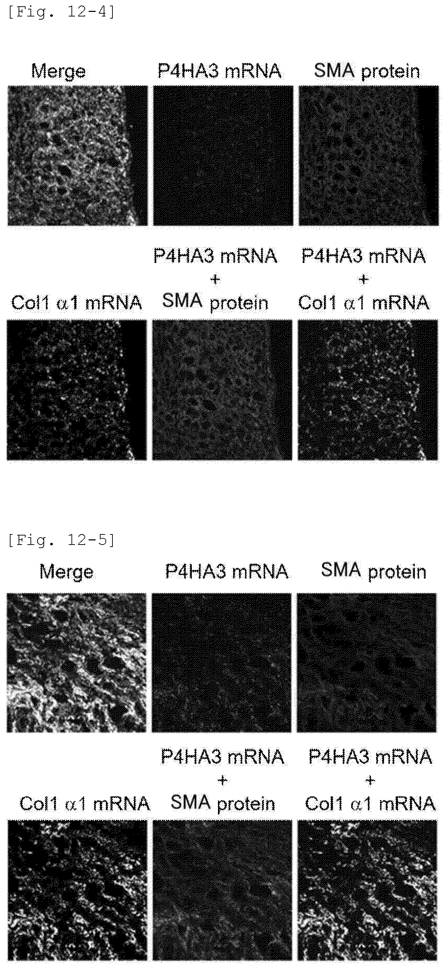

Fig. 12-4 shows fluorescent microscopy images of renal tissue sections from the UUO-2 region (see area ii in Fig. 12-1) on the side where unilateral ureteral obstruction was performed, showing the results of in situ hybridization using P4HA3 and 1α1 collagen probes, and fluorescent histochemistry using anti-α-SMAantibody (all at 20x magnification). The explanation for each image is the same as for Fig. 12-2. The UUO-2 region is also the region with the strongest expression oφ α-SMA, indicating that fibrosis is highly advanced.

Fig. 12-5 shows fluorescent microscopic images of renal tissue sections from the UUO-3 region (see area iii in Fig. 12-1) on the side where unilateral ureteral obstruction was performed, showing the results of in situ hybridization using P4HA3 and 1α1 collagen probes, and fluorescent immunohistochemistry using anti-α-SMAantibodies (all at 20x magnification). The explanation for each image is the same as for Fig. 12-2. The UUO-3 region is the area with weak to moderate expression of α-SMA, indicating that fibrosis is currently in progress.

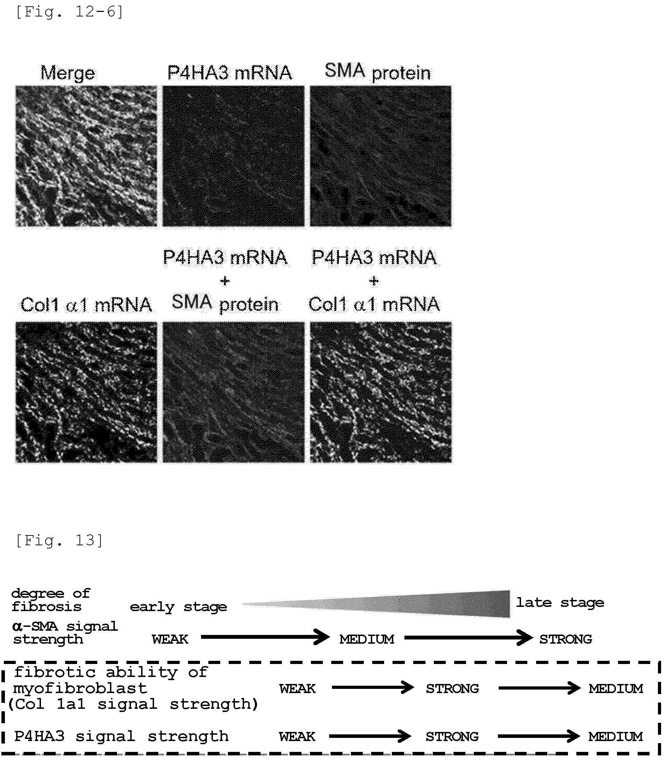

Fig. 12-6 shows fluorescent microscopic images of renal tissue sections from the UUO-5 region (see area v in Fig. 12-1) on the side where unilateral ureteral obstruction was performed, showing the results of in situ hybridization using P4HA3 and 1α1 collagen probes, and fluorescent histochemistry using anti-α-SMAantibody (all at 20x magnification). The explanation for each image is the same as in Fig. 12-2. The UUO-5 region is also the area with weak to moderate expression of α-SMA, indicating that fibrosis is currently in progress.

Fig. 13 is a schematic diagram summarizing the results obtained in Figs. 12-2 to 12-6. As the degree of fibrosis progressed from the early stage to the late stage, the expression of α-SMA, a marker for myofibroblasts, increased from weak to moderate to strong. However, it was revealed that 1α1 collagen, which reflects the fibrotic ability of myofibroblasts, and P4HA3 are most strongly expressed in the middle stage of fibrosis, and in the later stage of fibrosis, their expression decreases compared to the middle stage, but is higher than in the early stage of fibrosis, i.e., their expression is at a moderate level.

### Example 12: Liver fibrosis in P4HA3 gene-deficient mice

Next, liver fibrosis was examined in mice homozygous for the P4HA3 gene deficiency (hereinafter referred to as "P4HA3 knockout mice").

The P4HA3 knockout mice used in the examples of the present specification were created in the inventors' laboratory using the GONAD (Genome-editing via Oviductal Nucleic Acids Delivery) method (Ohtsuka, M. et al., Genome Biol. 19, 25 (2018)) in which a genome editing construct was constructed using the CRISPR/Cas9 system to prevent transcription of normal P4HA3 mRNA, and the construct was injected into the oviduct of a female C57BL/6N mouse the day after mating with a male C57BL/6N mouse, followed by electroporation of the entire oviduct.

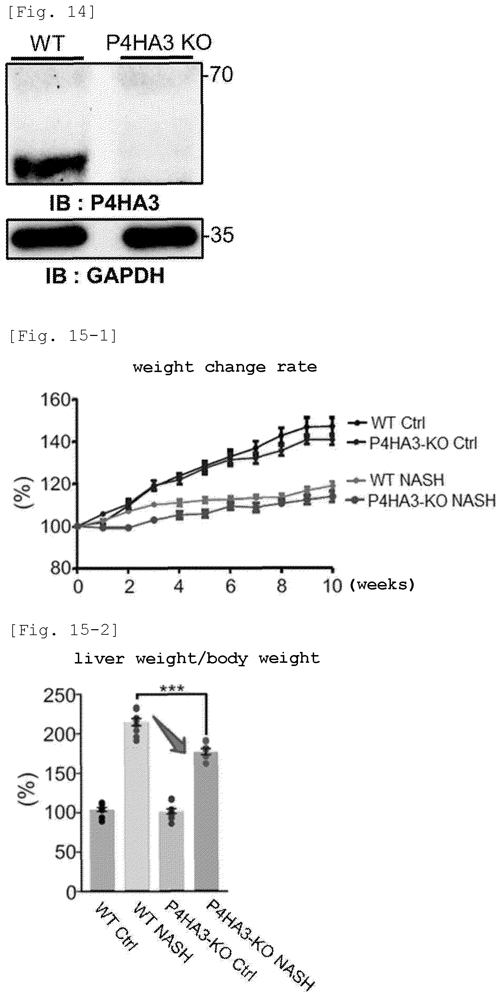

Fig. 14 is a Western blotting image showing the expression of P4HA3 protein in P4HA3 gene-deficient mice (P4HA3-KO) and wild-type mice (WT). The upper figure shows the results of polyacrylamide gel electrophoresis of total protein of hepatic myofibroblasts, which was detected with an antibody against P4HA3 protein, and the lower figure shows the results of the above-mentioned separation with an antibody against GAPDH protein. The 35 Kd band of the anti-GAPDH antibody was almost the same intensity in wild-type mice and P4HA3 knockout mice, whereas the 61 Kd P4HA3 protein was detected only in wild-type mice. Therefore, it was confirmed that the P4HA3 knockout mice of this example do not express P4HA3 protein. The creation of the NASH disease model by feeding an ultra-high fat, choline-deficient, methionine-reduced diet was performed in the same manner as in Example 4.

Fig. 15-1 is a line graph showing the time course of the weight change rate when P4HA3 gene-deficient mice (P4HA3-KO) and wild-type mice (WT) were fed an ultra-high fat, choline-deficient, methionine-reduced diet. From the top, the top line graph shows wild-type mice fed a normal diet (WT Ctrl), P4HA3 gene-deficient mice fed a normal diet (P4HA3-KO Ctrl), wild-type mice fed an ultra-high fat, choline-deficient, methionine-reduced diet (WT NASH), and the bottom line graph shows P4HA3 gene-deficient mice fed an ultra-high fat, choline-deficient, methionine-reduced diet (P4HA3-KO NASH). Fig. 15-2 is a bar graph showing the percentage of liver weight relative to body weight after feeding an ultra-high fat, choline-deficient, methionine-reduced diet or a normal diet for 10 weeks. The bar graphs show, from the left, the percentage of liver weight relative to body weight for wild-type mice fed a normal diet (WT Ctrl), wild-type mice fed an ultra-high fat, choline-deficient, methionine-reduced diet (WT NASH), P4HA3 gene-deficient mice fed a normal diet (P4HA3-KO Ctrl), and, on the right, P4HA3 gene-deficient mice fed an ultra-high fat, choline-deficient, methionine-reduced diet (P4HA3-KO NASH). The vertical axis shows the relative liver weight/body weight of each mouse group when the liver weight/body weight of wild-type mice fed a normal diet is taken as 100%. The number of mice in each group ranged from 8 to 10. *** indicates P<0.001. P values were calculated using Tukey's test. Error bars indicate standard deviation.

As shown in Fig. 15, when NASH was developed by feeding an ultra-high fat, choline-deficient, methionine-reduced diet, neither P4HA3 knockout mice nor wild-type mice gained weight, and even after 10 weeks, their weights remained almost the same as at the start of the experiment. However, the relative liver weight normalized by body weight was significantly lower in P4HA3 knockout mice that developed NASH than in wild-type mice that developed NASH. This indicates that liver hypertrophy is suppressed in P4HA3 knockout mice that developed NASH compared to wild-type mice, even when NASH is developed.

Furthermore, the expression of fibrosis-related factors was examined in P4HA3 knockout mice that developed NASH by feeding an ultra-high fat, choline-deficient, methionine-reduced diet. The quantification of the mRNA expression levels of fibrosis-related factors was performed in the same manner as in Example 2.

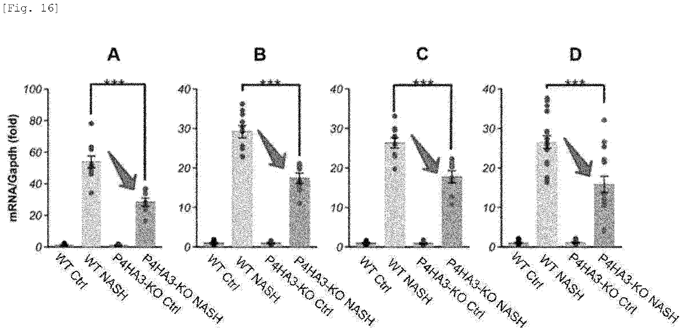

Fig. 16 is a bar graph comparing the relative expression levels of 1α1 collagen (A), 1α2 collagen (B), 3α1 collagen (C), and elastin (D) in P4HA3 gene-deficient mice and wild-type mice after 10 weeks of feeding a high-fat, choline-deficient, methionine-reduced diet or a normal diet. Each bar graph shows, from the left, the relative mRNA expression level of each gene in a wild-type mouse fed a normal diet (WT Ctrl), a wild-type mouse fed an ultra-high fat, choline-deficient, methionine-reduced diet (WT NASH), a P4HA3 gene-deficient mouse fed a normal diet (P4HA3-KO Ctrl), and, on the far right, a P4HA3 gene-deficient mouse fed an ultra-high fat, choline-deficient, methionine-reduced diet (P4HA3-KO NASH). The vertical axis shows the relative value of the mRNA expression level of each gene, where the value of the mRNA expression level of each gene in WT Ctrl corrected by the mRNA expression level of GAPDH is set to 1. The number of mice in each group ranged from 8 to 10. *** indicates P<0.001. P values were calculated using Tukey's test. Error bars indicate standard deviation.

As shown in Fig. 16, all fibrosis-related factors were significantly reduced in P4HA3 knockout mice that developed NASH compared to wild-type mice that developed NASH. This showed that liver hypertrophy was suppressed in P4HA3 knockout mice that developed NASH compared to wild-type mice. This demonstrated that P4HA3 is involved in promoting the expression of fibrosis-related factors in vivo as well.

Furthermore, the inventors histologically examined liver fibrosis in P4HA3 knockout mice that developed NASH due to feeding an ultra-high fat, choline-deficient, methionine-reduced diet. Mouse livers were immediately immersed in 10% neutral buffered formalin and fixed at room temperature for 16-32 hr, and paraffin-embedded thin sections were incubated in a picric acid-saturated 0.1% Direct Red 80 solution for 60 min and stained with Picrosirius Red. The sections were then washed with 0.01N HCl, dehydrated with ethanol, cleared with xylene, and mounted with MOUNT QUICK. Images of the stained liver tissue sections were taken under a microscope (KEYENCE, BZ-X700), and analyzed and quantified using KEYENCE Analysis Software.

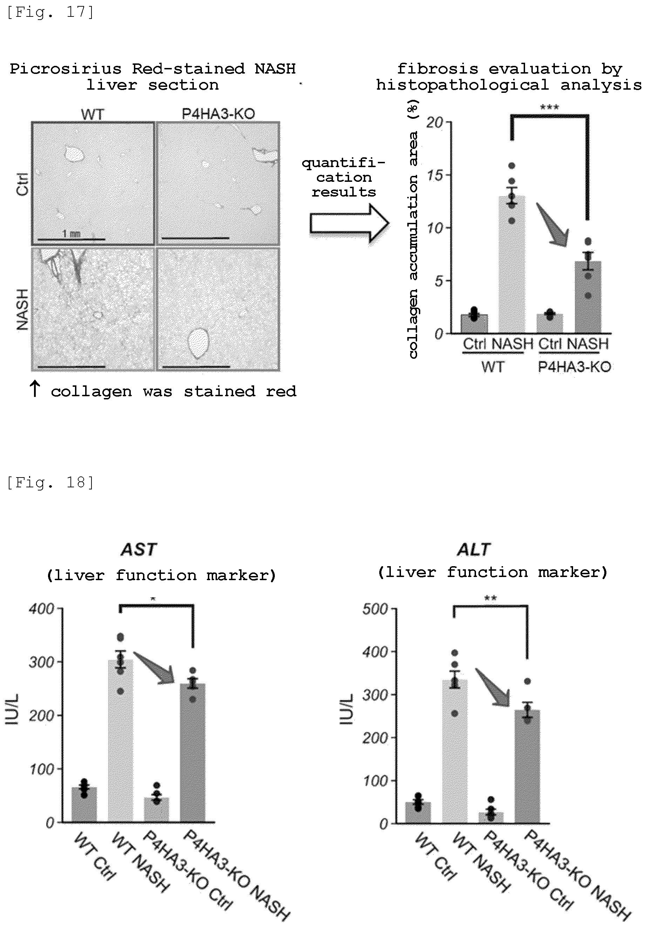

The left image in Fig. 17 shows Picrosirius Red stained optical microscope images of liver sections from P4HA3 gene-deficient mice (P4HA3-KO) or wild-type mice (WT) fed an ultra-high fat, choline-deficient, methionine-reduced diet (NASH) or a normal diet (Ctrl). The scale indicates 1 mm. The right image in Fig. 17 shows the evaluation results of histopathological analysis of these microscope images. Each bar graph shows, from the left, the percentage of collagen accumulation area in the liver of a wild-type mouse fed a normal diet (WT Ctrl), a wild-type mouse fed an ultra-high fat, choline-deficient, methionine-reduced diet (WT NASH), a P4HA3 gene-deficient mouse fed a normal diet (P4HA3-KO Ctrl), and, on the far right, a P4HA3 gene-deficient mouse fed an ultra-high fat, choline-deficient, methionine-reduced diet (P4HA3-KO NASH). The vertical axis of the graph shows the percentage of collagen accumulation area. There were six mice in each group. *** indicates P<0.001. P values were calculated using Tukey's test. Error bars show standard deviation.

As shown in the bar graph on the right of Fig. 17, the collagen accumulation area was significantly reduced in P4HA3 knockout mice with NASH compared to wild-type mice with NASH. Therefore, histological evaluation confirmed that the fibrotic pathology of NASH was improved in P4HA3 knockout mice.

Finally, the fibrosis of liver tissue in P4HA3 knockout mice with NASH caused by feeding an ultra-high fat, choline-deficient, methionine-reduced diet was examined by blood biochemistry. Mice were anesthetized with Somnopentyl, and whole blood was collected from the abdominal aorta using a syringe with a 25G needle. After that, the blood was centrifuged (1,200 x G, 5 min, 4°C) to separate the plasma from the serum. Plasma AST and ALT levels were measured at Unitech, and blood biochemistry tests were performed.

Fig. 18 is a bar graph showing the results of analysis of liver function markers AST (left) or ALT (right) in the serum of P4HA3 gene-deficient mice (P4HA3-KO) or wild-type mice (WT) fed an ultra-high fat, choline-deficient, methionine-reduced diet (NASH) or a normal diet (Ctrl). From the left, each bar graph shows the enzyme activity of AST or ALT in wild-type mice fed a normal diet (WT Ctrl), wild-type mice fed an ultra-high fat, choline-deficient, methionine-reduced diet (WT NASH), P4HA3 gene-deficient mice fed a normal diet (P4HA3-KO Ctrl), and on the far right, P4HA3 gene-deficient mice fed an ultra-high fat, choline-deficient, methionine-reduced diet (P4HA3-KO NASH). The vertical axis of the graph represents the enzyme activity of AST or ALT (unit: IU/L). The number of mice in each group was 5-6. The vertical axis represents the relative value of the mRNA expression level of each gene, with the GAPDH gene mRNA expression level taken as 1. * indicates P<0.05, ** indicates P<0.01. P values were calculated using Tukey's test. Error bars indicate standard deviation.

As shown in Fig. 18, the measured values of liver function markers AST and ALT were significantly lower in P4HA3 knockout mice that developed NASH than in wild-type mice that developed NASH. Therefore, blood biochemistry evaluation also confirmed that fibrotic pathology in NASH was improved in P4HA3 knockout mice.

### Example 13: Comparison of the effect of P4HA3 and Hsp47 knockdown on suppressing mRNA expression of fibrosis-related factors

In this example, the efficacy of P4HA3 knockdown treatment was compared with that of Hsp47 knockdown treatment, a candidate for treating liver fibrosis and cirrhosis that is already in phase II clinical trials.

Si Hsp47 and si P4HA3#1 from Example 7 were used as knockdown reagents for Hsp47 and P4HA3, respectively. For si Hsp47, ThermoFisher Scientific catalog number 4390771 (Assay ID: s232335) was used. Separation of cell type fractions from livers collected 4 weeks after the start of carbon tetrachloride administration was performed in the same manner as in Example 6. The quantification of mRNA expression levels of P4HA3 and fibrosis-related factors was carried out in the same manner as in Example 2. si Hsp47 and si P4HA3#1 were administered to cultured cells in the same manner as in Examples 3 and 7.

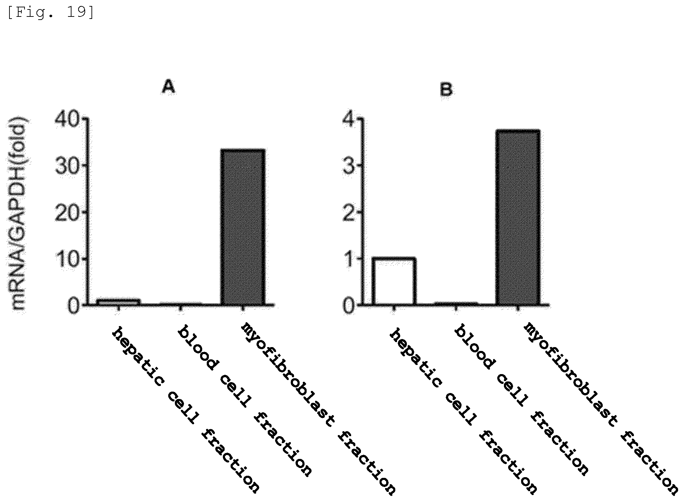

Fig. 19 is a bar graph showing the mRNA expression levels of each gene measured by real-time RT-PCR for each cell type fraction of the liver of a mouse model of transient liver fibrosis caused by carbon tetrachloride administration. Bar graph A shows the mRNA expression level of P4HA3, and bar graph B shows the mRNA expression level of Hsp47. The vertical axis shows the relative value of the mRNA expression level in each cell fraction, where the value obtained by correcting the mRNA expression level of each gene in the hepatic cell fraction by the mRNA expression level of GAPDH is taken as 1. The left of each bar graph shows the gene expression level in the hepatic cell fraction, the center shows the blood cell fraction, and the left shows the myofibroblast fraction.

As shown in Fig. 19, P4HA3 was expressed almost exclusively in the myofibroblast fraction, while Hsp47 was highly expressed in the myofibroblast fraction, but was also expressed in the hepatocyte fraction at an expression level that was just over 25% of that in the myofibroblast fraction. This demonstrates that P4HA3 has higher expression specificity in myofibroblasts than Hsp47.

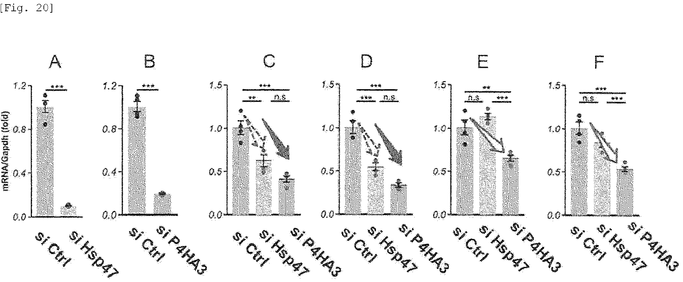

In Fig. 20, A is a bar graph showing the relative expression levels of the Hsp47 gene in myofibroblasts derived from fibrotic livers induced by carbon tetrachloride which were administered with si P4HA3 or si Ctrl. B is a bar graph showing the relative expression levels of the P4HA3 gene in myofibroblasts administered with si P4HA3 or si Ctrl. C to F are bar graphs showing the relative expression levels of 1α1 collagen (C), 3α1 collagen (D), 8α1 collagen (E), and 14α1 collagen (F) genes in myofibroblasts administered with si Ctrl, si Hsp47, or si P4HA3, respectively. The vertical axis shows the relative expression level of the mRNA of each gene, where the value obtained by correcting the mRNA expression level of each gene by the mRNA expression level of GAPDH is taken as 1. There are 4 mice in each mouse group. ** indicates P<0.01, *** indicates P<0.001, and n.s indicates no significant difference. P values were calculated by Tukey's test. Error bars indicate standard deviation.

As shown in Figs. 20A and B, si Hsp47 used in this example had a stronger effect of suppressing the mRNA expression of each target gene in myofibroblasts than si P4HA3. However, as shown in graphs C to F in Fig. 20, si P4HA3 had a stronger effect of suppressing the mRNA expression of fibrosis-related factors. The results in Fig. 20 therefore suggest that P4HA3 may have a more significant effect in suppressing fibrosis than the nucleic acid drug targeting Hsp47 currently under development.

Furthermore, the inventors investigated whether a synergistic effect on fibrosis inhibition could be obtained by using P4HA3 and Hsp47 siRNA in combination in myofibroblasts.

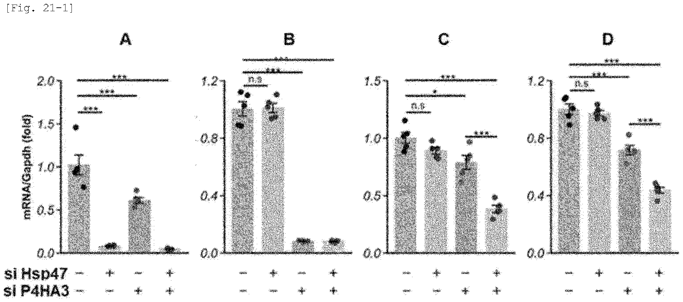

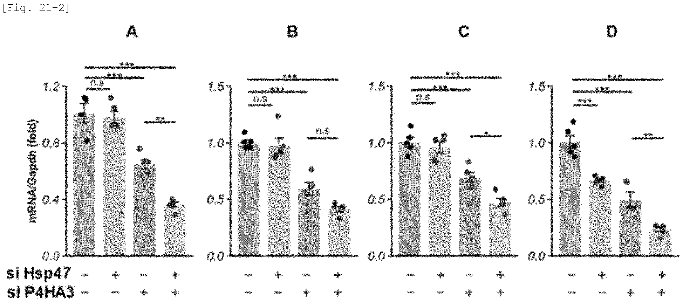

Graphs A to D in Fig. 21-1 are bar graphs showing the mRNA expression levels of Hsp47 (A), P4HA3 (B), 1α1 collagen (C), or 1α2 collagen (D) in myofibroblasts derived from damaged fibrotic livers administered with si Hsp47 and/or si P4HA3. Graphs A to D in21-2 are bar graphs showing the expression levels of 3α1 collagen (A), 8α1 collagen (B), 14α1 collagen (C), or elastin (D) genes in myofibroblasts derived from damaged fibrotic livers administered with si Hsp47 and/or si P4HA3. InFig. 21-1 and 21-2, the vertical axis shows the relative value of the mRNA expression level of each gene, with the mRNA expression level of GAPDH taken as 1. The results are from four experiments conducted under the same conditions. * indicates P<0.05, ** indicates P<0.01, *** indicates P<0.001, and n.s. indicates no significant difference. P values were calculated using Tukey's test. Error bars indicate standard deviation.

As shown in A and B of Fig. 21-1, Hsp47 siRNA did not suppress P4HA3 mRNA expression at all, but P4HA3 siRNA also slightly suppressed Hsp47 mRNA expression. As shown in C and D of Fig. 21-1 and A to D of 21-2, P4HA3 siRNA had a higher effect of suppressing the mRNA expression of fibrosis-related factors than Hsp47 siRNA, and it was revealed that the combined use of P4HA3 siRNA and Hsp47 siRNA had an even higher effect of suppressing the mRNA expression of fibrosis-related factors than either siRNA alone.

The above examples demonstrate that the P4HA3 gene is expressed only during fibrosis in the liver and other organs and is not expressed under normal conditions, that the P4HA3 gene is specifically expressed in myofibroblasts, which are the cells responsible for fibrosis in the liver and other organs, and that expression of P4HA3 promotes the expression of fibrosis-related factors, thereby worsening the fibrotic pathology.

### Example 14: P4HA3 as a poor prognostic factor for cancer

Fibrotic diseases include not only fibrotic diseases of organs such as myocardial infarction, cirrhosis, and pulmonary fibrosis, but also cancers accompanied by physical hardening of the lesions. Therefore, the possibility that expression of the P4HA3 gene is a poor prognostic factor for cancer was examined.

The Kaplan-Meier Plotter (Gyoerffy, B., Computational and Structural Biotechnology Journal, 2021; 19:4101-4109), available online (http://kmplot.com/analysis/index.php?p=service&cancer=pancance r_rnaseq), contains data on the expression of 54,000 types of genes (mRNA, miRNA, protein) and prognostic information (Kaplan-Meier curves) for 21 types of cancer. The Kaplan-Meier Plotter was used to search for cancers in which the expression level of the P4HA3 gene is related to prognosis.

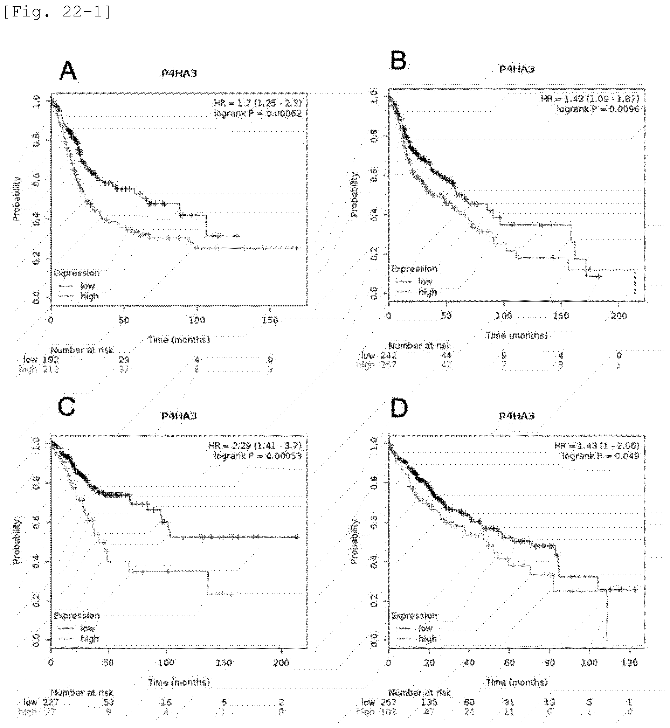

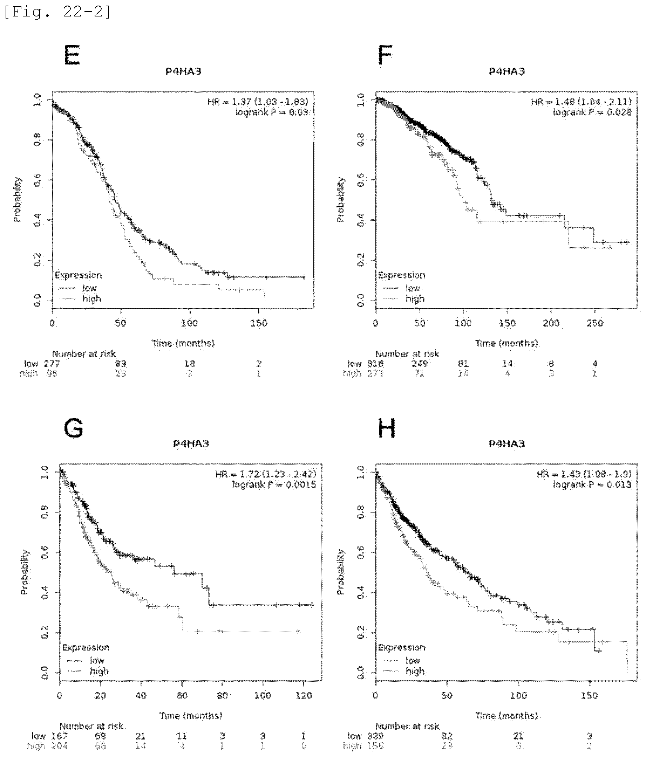

Figs. 22-1 to 22-3 are Kaplan-Meier curves showing the relationship between the prognosis of bladder cancer (A), head and neck squamous cell carcinoma (B), cervical squamous cell carcinoma (C), liver hepatocellular carcinoma (D), pancreatic ductal carcinoma (E), breast cancer (F), gastric adenocarcinoma (G), lung squamous cell carcinoma (H), rectal adenocarcinoma (I), and ovarian cancer (J) and the level of P4HA3 gene expression. Cases with high P4HA3 gene expression are shown in black, and cases with low P4HA3 gene expression are shown in gray. Both were created using Kaplan-Meier Plotter.

As shown in Figs. 22-1 to 22-3, cases with high P4HA3 gene expression had a worse prognosis than cases with low expression of the gene in all cancers. Liu, M. et al. (Scientific Reports volume 10, Article number: 12949 (2020)) reported that P4HA3 gene expression was higher in all 15 cases of renal clear cell carcinoma examined than in the corresponding normal renal tissues. Since P4HA3 siRNA strongly suppressed the mRNA expression of not only the P4HA3 gene but also other fibrosis-related factors in myofibroblasts, it is predicted that P4HA3 siRNA is likely to be effective in cancer cells as well.

### Example 15: P4HA3 isoforms

In addition to P4HA3, there are also alpha 1 subunit (P4HA1) and alpha 2 subunit (P4HA2) in the alpha subunit of prolyl 4-hydroxylase. The inventors therefore investigated whether there are differences in gene expression and expressing cells between these isoforms.

Table 1 shows the TPM (transcripts per million) values of P4HA3, P4HA1, and P4HA2 in single-cell RNA libraries derived from tissues collected from sham-treated hearts (Sham), the infarcted region of treated hearts (Inf), the border region of the infarcted region of treated hearts (Border), and the region distant from the infarcted region of treated hearts (Rem) at 3 days (3d), 7 days (7d), and 14 days (14d) after myocardial infarction or sham treatment, based on the data of Farbehi N. et al. (2019) mentioned above.

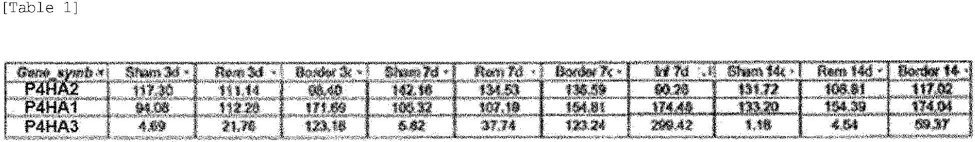

As shown in Table 1, the number of TPMs of P4HA3 was significantly higher in the infarcted area 7 days after myocardial infarction (Inf 7d), but the change in the number of TPMs of P4HA1 and P4HA2 did not increase in the infarcted area and was not associated with myocardial infarction treatment. This result suggests that only P4HA3, among the isoforms of the alpha subunit of prolyl 4-hydroxylase, is specifically increased in myocardial infarction.

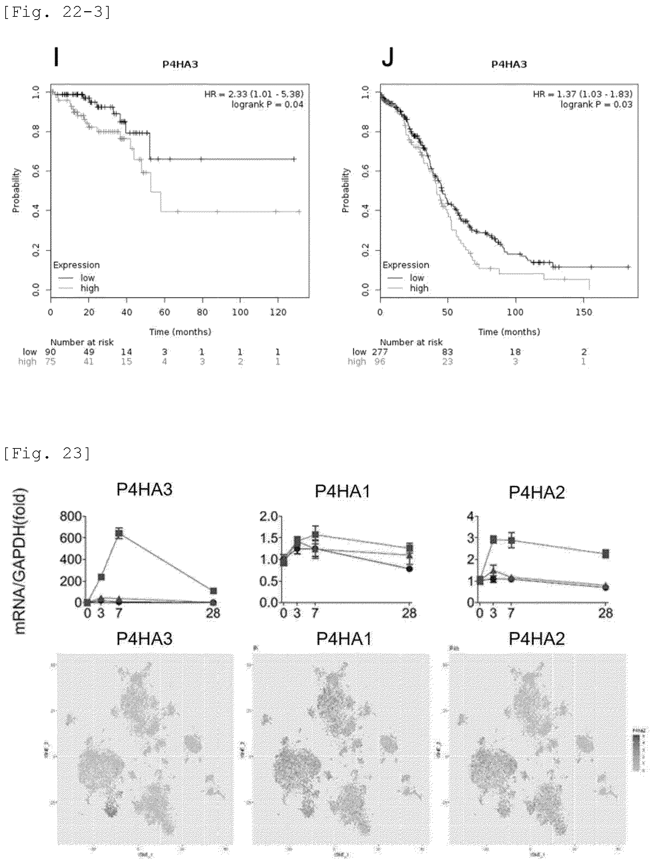

The upper part of Fig. 23 is a graph showing the time course of the expression levels of P4HA3, P4HA1, and P4HA2 in the heart after myocardial infarction. ■ indicates the mRNA expression level of each gene measured by real-time RT-PCR in the infarcted area, ▲ indicates the mRNA expression level of each gene measured by real-time RT-PCR in the non-infarcted area, and ● indicates the mRNA expression level of each gene measured by real-time RT-PCR in the sham treatment group. The vertical axis represents the relative value of the mRNA expression level of each gene, with the GAPDH mRNA expression level taken as 1, and the horizontal axis represents the number of days after myocardial infarction treatment (unit: days). The lower part of Fig. 23 is a t-SNE plot created using the data of Farbehi N. et al. (2019) mentioned above, which is a dimension reduction plot that visualizes cell populations expressing P4HA3, P4HA1, and P4HA2.

As shown in the upper part of Fig. 23, P4HA3 mRNA expression in the infarcted area increases immediately after the onset of myocardial infarction, reaches a maximum after 7 days, and almost disappears after 28 days. However, P4HA3 mRNA was not detected at all in the non-infarcted area or in the sham treatment group. In contrast, P4HA1 mRNA was expressed almost constantly in both the treatment group and the sham treatment group, regardless of the onset of myocardial infarction. P4HA2 mRNA increased in the infarcted area immediately after the onset of myocardial infarction, reaching a peak 7 days later, but was still strongly expressed 28 days later. In the non-infarcted area and sham treatment group, expression remained almost constant at the same level as at the onset of myocardial infarction.

As shown in the bottom row of Fig. 23, P4HA3 mRNA was expressed almost exclusively in myofibroblasts, while P4HA1 mRNA was expressed not only in myofibroblasts but also in fibroblasts, macrophages, and endothelial cells, and P4HA2 mRNA was expressed not only in myofibroblasts but also in fibroblasts and endothelial cells.

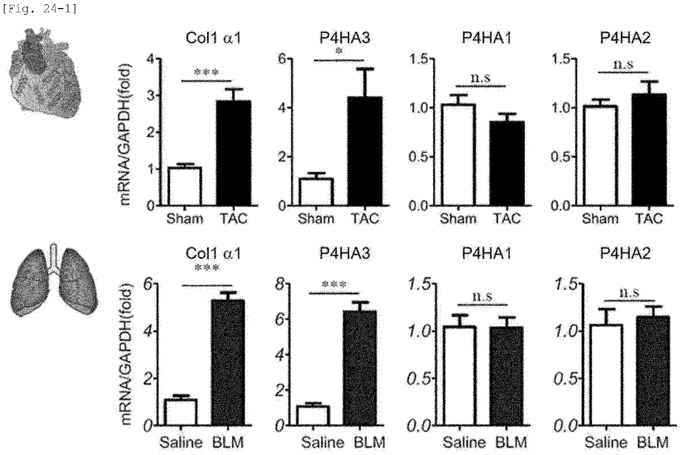

The top of Fig. 24-1 is a bar graph showing the expression levels of 1α1 collagen gene (Col1α1) and the mRNAs of different isoforms of P4HA3 mRNA, P4HA3, P4HA1, and P4HA2, in cardiac tissue from myocardial infarction induced by transverse aortic coarctation (TAC) or cardiac tissue from sham-treated control mice (Sham). The bottom of Fig. 24-1 is a bar graph showing the expression levels of 1α1 collagen mRNA (Col1α1) and the mRNAs of P4HA3, P4HA1, and P4HA2, in lung tissue from bleomycin-administered lung fibrosis model mice (BLM) or lung tissue from saline-administered control mice (Saline).

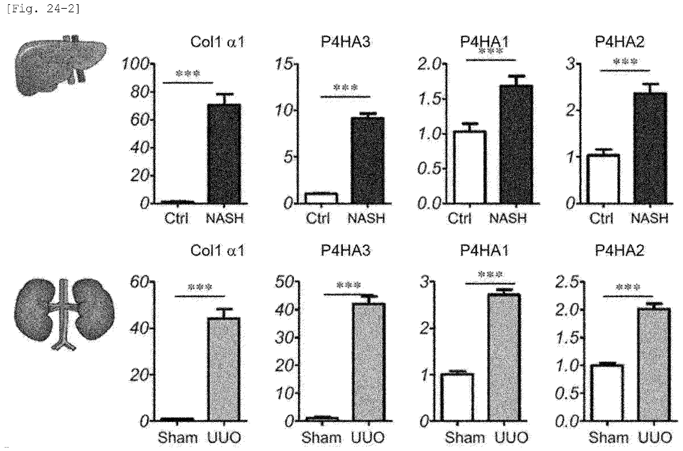

The top of Fig. 24-2 is a bar graph showing the mRNA expression levels of 1α1 collagen gene (Col1α1) and different P4HA isoforms P4HA3, P4HA1, and P4HA2 in liver tissues of mice that developed NASH by feeding an ultra-high fat, choline-deficient, methionine-reduced diet (NASH) or in liver tissues of control mice fed a normal diet (Ctrl). The bottom of Fig. 24-2 is a bar graph showing the mRNA expression levels of 1α1 collagen mRNA (Col1α1) and different P4HA isoforms P4HA3, P4HA1, and P4HA2 in kidney tissues of renal fibrosis model mice induced by unilateral ureteral obstruction (UUO) or in kidney tissues of sham-treated control mice (Sham). In Figs. 24-1 and 24-2, the vertical axis represents the relative value of the mRNA expression level of each gene, with the mRNA expression level of GAPDH set to 1. * indicates P<0.05, *** indicates P<0.001, and n.s. indicates no significant difference. P values were calculated using Student's t-test. Error bars indicate standard deviation.

As shown in Figs. 24-1 and 24-2, the mRNA expression level of P4HA3 in the treatment group was 4 to 40 times higher than that of the sham treatment group. However, the mRNA expression levels of P4HA1 and P4HA2 in the treatment group were only 0.8 to 4.5 times higher than that of the sham treatment group. Therefore, it was confirmed that even though they are the same P4HA alpha subunit isoforms, only P4HA3 was specifically expressed in the treatment group in response to the mRNA expression of fibrosis-related factors.

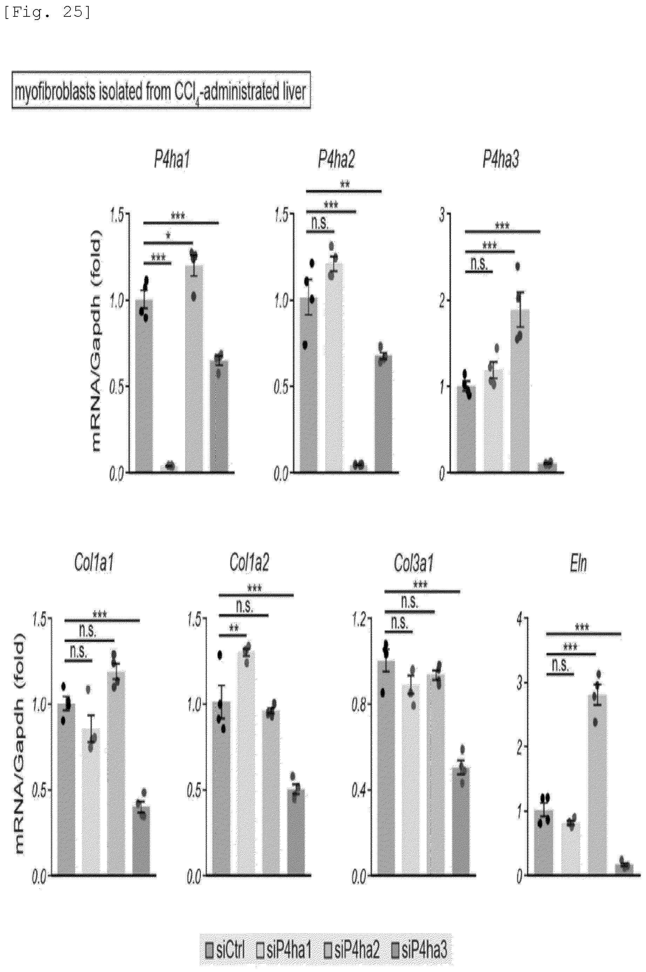

Fig. 25 is a bar graph showing the effects of si Ctrl, si P4HA1, si P4HA2, and si P4HA3 on gene expression of fibrosis-related factors (P4HA1, P4HA2, P4HA3, 1α1 collagen (Col1α1), 1α2 collagen (Col1α2), 3α1 collagen (Col3α1), and elastin (Eln)) in myofibroblasts isolated from liver tissue of a mouse model of liver damage and fibrosis caused by carbon tetrachloride administration. The vertical axis shows the relative value of the mRNA expression level of each gene, where the value obtained by correcting the mRNA expression level of each gene in the si Ctrl group by the mRNA expression level of GAPDH is set to 1. The number of samples for each group was 4. Comparisons between groups were calculated using one-way analysis of variance followed by Tukey's range test. * indicates P<0.05, ** indicates P<0.01, *** indicates P<0.001, and n.s. indicates no significant difference. Error bars indicate standard deviation.

From Fig. 25, si P4HA1, si P4HA2, and si P4HA3 almost completely suppressed the expression of P4HA1, P4HA2, and P4HA3 genes, respectively.

### Example 16: Relationship between TGFβ/SMAD3 pathway involved in liver fibrosis and P4HA3

Hepatic stellate cells, which are involved in liver fibrosis, are known to be activated by TGFβ (Fabregat, I. et al., FEES J., 283: 2219-2232 (2016)). Therefore, the effects of TGFβ and its specific inhibitor SIS3, as well as an inhibitor of SMAD3 involved in signal transduction of the TGFβ pathway (si SMAD3), on the mRNA expression of fibrosis-related factors in NASH model mice were examined. SIS3 (CAS number: 1009104-85-1) was used under catalog number 15945 from Cayman Chemical Company. SIS3 was added to the culture medium at a final concentration of 3 µM and administered. Si SMAD3 was used as a knockdown reagent for SMAD3. si SMAD3 was used under catalog number 4390771 (Assay ID: s69495) from ThermoFisher Scientific, si SMAD3 was administered to cultured cells in the same manner as in Examples 3, 7, and 13. LX-2, a cultured hepatic stellate cell line, was cultured in DMEM supplemented with 2% FBS.

First, the effect of TGFβ on the mRNA expression of P4HA3 or 1α1 collagen (Col1α1) in the cultured LX-2 cells was examined. Fig. 26a is a bar graph showing the mRNA expression level of P4HA3 or 1α1 collagen (Col1α1) in LX-2 cells treated with DMSO or TGFβ (5 ng/mL) for 12 hr. The vertical axis shows the relative value of the mRNA expression level of each gene, where the value obtained by correcting the mRNA expression level of each gene in the Ctrl group by the mRNA expression level of GAPDH is set to 1. The number of samples for each group was 3. Comparisons between groups were calculated by one-way analysis of variance followed by Tukey's range test. * indicates P<0.05, ** indicates P<0.01, *** indicates P<0.001, and n. s indicates no significant difference. Error bars indicate standard deviation. As shown in Fig. 26a, the mRNA expression of P4HA3 or 1α1 collagen (Col1α1) is enhanced by the addition of TGFβ.

Bar graph showing the mRNA expression levels of P4HA3, 1α1 collagen (Col1α1),1α2 collagen (Col1α2), and 3α1 collagen (Col3α1) in myofibroblasts isolated from liver tissue of a mouse model of liver damage and liver fibrosis caused by carbon tetrachloride administration and treated with SIS3, an inhibitor of the TGFβ/SMAD pathway. The vertical axis shows the relative value of the mRNA expression level of each gene, where the value corrected by the mRNA expression level of each gene in the si Ctrl group by the mRNA expression level of GAPDH is set to 1. The number of samples for each group was 5. Comparisons between groups were calculated using one-way analysis of variance followed by Tukey's range test. * indicates P<0.05, ** indicates P<0.01, *** indicates P<0.001, and ns indicates no significant difference. As shown in Fig. 26b, SIS3 suppressed the mRNA expression of fibrosis-related factors including P4HA3.

Fig. 26c is a bar graph showing the mRNA expression levels of SMAD3, P4HA3, 1α1 collagen (Col1α1),1α2 collagen (Col1α2), and 3α1 collagen (Col3α1) in myofibroblasts isolated from liver tissue of a mouse model of liver damage and liver fibrosis caused by carbon tetrachloride administration and transfected with siRNA against SMAD3 (si Smad3) or si Ctrl. The vertical axis shows the relative value of the mRNA expression level of each gene, where the value obtained by correcting the mRNA expression level of each gene in the si Ctrl group by the mRNA expression level of GAPDH is taken as 1. The number of samples for each group was 5. Comparisons between groups were calculated by one-way analysis of variance followed by Tukey's range test. * indicates P<0.05, ** indicates P<0.01, *** indicates P<0.001, and n.s indicates no significant difference. As shown in Fig. 26c, si Smad3, which inhibits the mRNA expression of SMAD3, also suppressed the mRNA expression of fibrosis-related factors including P4HA3.

Therefore, it was revealed that P4HA3 according to the present invention is regulated by the TGFβ/SMAD3 pathway, like other fibrosis-related factors.

This application is based on Japanese Patent Application No. 2022-032915 filed in Japan on March 3, 2022, the entire contents of which are incorporated herein by reference.

### [Industrial Applicability]

The present invention makes it possible to improve fibrotic diseases through the suppression of the mRNA expression of fibrosis-related factors. In addition, by using it in combination with a therapeutic drug for fibrosis diseases that has already been developed, such as Hsp47, it becomes possible to further improve fibrotic diseases.

## Claims

1. A pharmaceutical composition for treating or preventing a fibrotic disease, comprising a nucleic acid that inhibits expression of mRNA encoding the alpha 3 subunit of prolyl 4-hydroxylase (P4HA3).

2. The pharmaceutical composition according to claim 1, wherein the fibrotic disease is at least one disease selected from the group consisting of fibrosis, liver cirrhosis, renal failure, myocardial infarction, and cancer.

3. The pharmaceutical composition according to claim 1 or 2, wherein the nucleic acid has a nucleotide sequence that is complementary to at least a portion of the mRNA encoding P4HA3.

4. The pharmaceutical composition according to any one of claims 1 to 3, wherein the nucleic acid is an siRNA or an antisense nucleic acid.

5. The pharmaceutical composition according to claim 4, wherein the siRNA is an siRNA consisting of an oligonucleotide comprising at least 15 consecutive sequences of any one of the sequences of SEQ ID NOs: 9 to 11, and an oligonucleotide comprising a sequence complementary to at least 15 consecutive sequences of the oligonucleotide.

6. The pharmaceutical composition according to claim 4, wherein the siRNA is a pair of an oligonucleotide comprising the sequence of SEQ ID NO:5 and an oligonucleotide comprising the sequence of SEQ ID NO:6, or a pair of an oligonucleotide comprising the sequence of SEQ ID NO:7 and an oligonucleotide comprising the sequence of SEQ ID NO:8.

7. The pharmaceutical composition according to claim 5, wherein the siRNA is a pair of an oligonucleotide consisting of the sequence of SEQ ID NO:1 and an oligonucleotide consisting of the sequence of SEQ ID NO:2, or a pair of an oligonucleotide consisting of the sequence of SEQ ID NO:3 and an oligonucleotide consisting of the sequence of SEQ ID NO:4.

8. The pharmaceutical composition according to any one of claims 1 to 7, comprising a liposome that specifically adsorbs to myofibroblasts, the liposome encapsulating the nucleic acid.
